# EUROPEAN PATENT SPECIFICATION

(11) **EP 3 043 809 B1**
(45) Date of publication and mention of the grant of the patent: **31.01.2018**
(21) Application number: 14786320.3
(22) Date of filing: 11.09.2014
(51) Int. Cl.: A61K 36/73, A61P 35/00, A61P 37/00, A61K 31/519, A61K 31/282, A61K 31/337, A61K 31/704

(54) **FILIPENDULA VULGARIS EXTRACT AND USES THEREOF**
FILIPENDULA-VULGARIS-EXTRAKT UND VERWENDUNGEN DAVON
EXTRAIT DE FILIPENDULA VULGARIS ET UTILISATIONS DE CELUI-CI

(30) Priority: 11.09.2013 IT RM20130502
(43) Date of publication of application: 20.07.2016
(73) Proprietor: Aboca S.p.A. Società Agricola, 52037 Sansepolcro (AR) (IT)
(72) Inventor: MERCATI, Valentino, I-52037 Sansepolcro AR (IT); MAIDECCHI, Anna, I-52037 Sansepolcro AR (IT)
(74) Representative: Predazzi, Valentina
(86) International application number: PCT/IB2014/064422
(87) International publication number: WO 2015/036949

(56) References cited:
- Ilinca M Imbrea ET AL: "Determining antioxidant capacity of extracts of Filipendula vulgaris Moench from south-western Romania", Journal of Food Agriculture & Environmentnet Journal of Food Agriculture & Environment, 1 July 2010 (2010-07-01), pages 111-116, XP055113207, Retrieved from the Internet: URL:http://world-food.net/download/journal s/2010-issue_3_4/17.pdf [retrieved on 2014-04-09]
- OSZMIANSKI ET AL: "Antioxidant tannins from Rosaceae plant roots", FOOD CHEMISTRY, ELSEVIER LTD, NL, vol. 100, no. 2, 3 July 2006 (2006-07-03), pages 579-583, XP005852236, ISSN: 0308-8146, DOI: 10.1016/J.FOODCHEM.2005.09.086
- DATABASE WPI Week 200169 Thomson Scientific, London, GB; AN 2001-606057 XP002723095, & KR 2001 0035299 A (PARK K Y) 7 May 2001 (2001-05-07)
- DATABASE WPI Week 200158 Thomson Scientific, London, GB; AN 2001-528090 XP002723096, & KR 2001 0025574 A (PARK K Y) 6 April 2001 (2001-04-06)
- SYLVIA VOGL ET AL: "Ethnopharmacological in vitro studies on Austria's folk medicine-An unexplored lore in vitro anti-inflammatory activities of 71 Austrian traditional herbal drugs", JOURNAL OF ETHNOPHARMACOLOGY, 1 June 2013 (2013-06-01), XP055074668, ISSN: 0378-8741, DOI: 10.1016/j.jep.2013.06.007
- HUA YU ET AL: "STATs in cancer inflammation and immunity: a leading role for STAT3", NATURE REVIEWS CANCER, vol. 9, no. 11, 1 November 2009 (2009-11-01), pages 798-809, XP055113218, ISSN: 1474-175X, DOI: 10.1038/nrc2734
- AGGARWAL B B ET AL: "Inflammation and cancer: How hot is the link?", BIOCHEMICAL PHARMACOLOGY, ELSEVIER, US, vol. 72, no. 11, 30 November 2006 (2006-11-30), pages 1605-1621, XP027905314, ISSN: 0006-2952 [retrieved on 2006-11-30]

## Description

### TECHNICAL FIELD OF THE INVENTION

The present invention relates to an extract of *Filipendula vulgaris* according to Moench *(*synonym: *Filipendula hexapetala* Gilib.) and to compositions and kits that comprise said extract for use in the the prevention and/or the treatment of a pathological condition characterised by a constitutive activation of the STAT3 transcription factor.

### PRIOR ART

In recent decades, much evidence in literature indicates the fundamental role of transcription factors belonging to the STAT family in a wide variety of pathologies, such as in inflammatory pathologies that promote tumours, and in tumours themselves. STAT proteins are cytoplasmic transcription factors of which the phosphorylation/activation (on specific residues of serine and/or tyrosine due to the action of the families of JAK, or Janus kinase proteins) determines the dimerization of two STAT monomers, the translocation of the dimer in the nucleus, the binding to elements of the DNA of STAT-specific target genes, and the induction of gene transcription. The family of the STAT factors consists of seven members (coded by the genes STAT1, STAT2, STAT3, STAT4, STAT5A, STAT5B and STAT6) with various biological functions that include roles in differentiation, proliferation, development, apoptosis and cell inflammation. One characteristic of the proteins coded by these genes is that of having a dual role, more specifically a role of transduction of the signal in the cytoplasm and of transcription factor in the nucleus. In particular, a constitutive activation of STAT3 and to a lesser extent of STAT5 has been associated in various neoplasias with the deregulation of some intracellular pathways, including those involved in the survival of the tumour and in the proliferation of the tumour cell, but also in the process of angiogenesis and metastasis of the tumour itself.

YuH. et al in a review published on Nature in 2009 (Nature Reviews Cancer 9, 798-809: 2009) reported that the persistent activation of STAT3 induces inflammation that promotes the cancer and regulates genes crucial for the inflammation and the tumour microenvironment. Genes activated by STAT3 are shown in Tables 1 and 2 of the above-mentioned work, and some inhibitors of the activation of STAT3 are also described among natural substances, such as curcubitacin, resveratrol, galiellalactone and indirubin, however it is stated that the mechanisms of actions by which these substances act are unknown.

In any case, the work states that the modulation of STAT3 is a new, more effective and highly advantageous approach for treating cancer, reporting that the ablation of the STAT3 gene in various tumour models led to inhibition of tumour growth.

The constitutive activation of STAT3 has been reported in a large number of tumours, including breast cancer, prostate cancer, squamous-cell carcinoma of the head and neck, multiple myeloma, lymphoma and leukaemia, brain tumours, colon cancer, Ewing's sarcoma, stomach cancer, oesophageal cancer, ovarian cancer, nasopharyngeal cancer, and pancreatic cancer (Table 1 below). For many types of cancer, high levels of activated STAT3 have been linked to a poor prognosis. The activation of STAT3 blocks apoptosis and increases cell proliferation and cell survival, promoting angiogenesis and metastasis and inhibiting the anti-tumour immune responses. Tumour cell lines in which STAT3 is constitutively activated require the continuous activation of STAT3, a phenotype that has been defined as "dependence on oncogenes" (Johnston PA and Grandis RG, Mollnterv; 11 (1); 18-26:2011).

Malignant plural mesothelioma (MPM) is an aggressive tumour derived from the mesothelial cells of the chest cavities, and, although chemotherapy (often if pemetrexed is used) improves the survival time in patients with non-operable MPM, the average global survival time is just 12 months. It has been reported recently that a potential molecular therapeutic target for MPM is the interleukine-6 signalling pathway (IL-6)/JAK/STAT3 activated by the high level of IL-6 present in pleural liquid of patients with MPM. The bind of IL-6 to its receptor causes a conformational change in the receptor that initiates JAK activation, which in turn initiates the dimerization of the STAT3 transcription factor, and the STAT3 dimer translocates in the nucleus, thus determining the initiation of the transactivation of various target genes.

This pathway is key for the occurrence of haematopoiesis, of the immune response and of oncogenesis. In addition, it has also been demonstrated that the dysfunction of the JAK/STAT3 system is involved in the development of cancer.

In addition, a broad description of the role played by STAT3 in the development and in the progression of the tumour is ever present in the literature. A constitutive activation of STAT3 has been observed both in blood tumours (multiple myeloma, leukaemia, lymphoma) and in solid tumours (melanomas, carcinoma of the ovaries, of the prostate and of the renal cells, pancreatic adenocarcinoma, lung cancer and brain cancer). For greater depth, Table 3 below, taken from Turkson J and Jove R, Oncogene; 19(56);6613-26: 2000, indicates numerous tumours directly associated with the anomalous activation of STAT3. In particular, this anomalous activation seems to be caused by the action of transforming tyrosine kinases, such as v-Src, v-Ros, v-Fps, Etk/BMX and Lck, or by an anomalous signal induced by the autocrine or paracrine release of cytokines. The constitutive activation of STAT3 leads to a greater expression of genes coding for inhibitors of apoptosis (for example Bcl-xL, Mcl-1), regulators of the cell cycle (for example cyclin D1/D2, c Myc) and inducers of angiogenesis (for example VEGF: Vascular Endothelial Growth Factor). Lastly, it has been demonstrated recently that apart from having a key role in tumourigenesis, the constitutive activation of this transcription factor confers resistance to the death induced by chemotherapeutic agents (Aggarwal B. B. et al. Ann. N.Y. Acad. Sci. 1091; 151-69: 2006).

A variety of clinical research has demonstrated that, *in vivo,* solid tumours grow and develop in an environment with low levels of O₂ that make the tumour itself insensitive to the signals of cell death and resistant to radiotherapy and chemotherapy treatments; on the other hand, the hypoxia promotes angiogenesis, proliferation and metastatic ability. The aggressiveness of the tumour in this context seems to be associated with the activation and stabilisation of the factor of HIF-1α both by the hypoxia and by the hyperactivation of STAT3.

For this reason, an anti-cancer therapy based on the targeting of the factor STAT3 is highly desirable (Niu G. et al. Mol Cancer Res, 6 (7); 1099-105: 2008).

Table 1, shown below, is taken from the work of Aggarwal B. B. et al. 2006 and shows a list of tumours that express constitutively active STAT3, activators of STAT3, genes regulated by STAT3 and inhibitors of STAT3

**Table 1**

| **Constitutive STAT3** | **Activators** | **Genes** | **Kinases** | **Inhibitors** |
|---|---|---|---|---|
| **Haematopoietic tumours** | EGF | **Antiapoptosis** | **Non-receptor tyrosine kinases** | **Synthetic** |
| -Multiple myeloma | IL-6 | Bcl-X_{L} | | AG490 |
| -HTLV-1-dependent | IL-5 | Bcl-2 | JAK | Sodium salicylate |
| leukaemia | IL-9 | Mcl-1 | JAK2 | Atiprimod |
| -CLL | IL-10 | clAP-2 | JAK3 | BMS-354825 |
| -CML | IL-12 | Survivin | TYK2 | Ethanol |
| -AML | IL-22 | | Src | Nelfinavir |
| -Large granular lymphocyte | TNF-α | **Cell cycle progression** | | PS-341 |
| leukaemia | MCP-1 | | **Receptor tyrosine kinases** | R115777 |
| -Erythroleukaemia | GCSF | Cyclin D1 | | WP-1034 |
| -Polycythaemia vera | GMCSF | c-Myc | EGFR | Platinum compounds |
| -EBV-related/Burkitt's | CSF | c-Fos | ErbB-2 | 15-Deoxy-delta |
| -Mycosis fungoides | LIF | p21 | Gp130 | 12,14-PGJ2 |
| -Cutaneous T cell lymphoma | OSM | | Grb2 | UCN-01 |
| -HSV saimiri-dependent (T cell) | IFN-γ | **Tumour invasion and metastasis** | | Statin |
| | MIP-1α | | **Serine kinases** | |
| -Hodgkin's disease | RANTES | MMP-2 | JNK | **Peptides** |
| -Anaplastic lymphoma | SLF | MMP-9 | P38MAPK | SOCS3 |
| | UVB | β-catenin | ERK | PIAS |
| **Solid tumours** | Osmotic shock | VEGF | | GRIM-19 |
| -Breast cancer | Progestin | hTERT | **Tyrosine** | Adiponectin |
| -Brain tumour | LPS | | | Duplin SSI-1 |
| -Colon carcinoma | Tobacco | IRF-1NLK | **phosphatase** | |
| -Ewing's sarcoma | HCV | MyD88 | | α-Thrombin |
| -Gastric carcinoma | | RANKL | | Lipoxin A4 |
| -Lung cancer | | TNF | | DIF-1 |
| -Nasopharyngeal cancer | | β-macroglobulin | | PTPεC |
| -Ovarian carcinoma | | SOCS | | STAT3-DN |
| -Pancreatic adenocarcinoma | | Angiotensinogen | | Decoy peptide |
| -Prostate carcinoma | | Antichymotrypsin | | |
| -Renal cell carcinoma | | | | **Naturals** |
| -SCCHN cancer | | | | Flavopiridol |
| | | | | Indirubin |
| | | | | Magnolol |
| | | | | Resveratrol |
| | | | | Piceatannol |
| | | | | Parthenolide |
| | | | | EGCG |
| | | | | Curcumin |
| | | | | Cucurbitacin |
| | | | | **Others** |
| | | | | Rituximab |
| | | | | GQ-ODN |
| | | | | Retinoic acid |
| | | | | STA-21 |
| | | | | EKB569 |

| | | | | |
|---|---|---|---|---|
| **Key:** STAT, signal-transducer-and-activator-of-transcription; CLL, chronic lymphocytic leukaemia; CML, chronic myeloid leukaemia; AML, acute myelogenous leukaemia; SCCHN, squamous cell carcinoma of the head and neck, HTLV, human T cell lymphotropic virus; EBV, Epstein-Barr virus; Nelfinavir, HIV-1 protease inhibitor; R115777, farnesyl transferase inhibitor; AG490 and piceatannol, tyrosine kinase inhibitors; PIAS, protein inhibitor of activated STAT3; GQ-ODN, G-quartet oligonucleotides; SOCS, suppressor of cytokine signalling; GRIM, gene associated with retinoid-IFN-induced mortality; EGCG, epigallocatechin-3-gallate; SSI, STAT-induced STAT inhibitor; PTPεC, protein tyrosine phosphatase εC; DN, dominant negative; EKb-569, EGF-R inhibitor; DIF-1, differentiation-inducing factor-1; JAB, SH2-domain-containing protein; IL, interleukin; TNF, tumour necrosis factor; MDA, melanoma differentiation antigen; MCP, monocyte chemoattractant protein; GCSF, granulocyte colony-stimulating factor; LIF, leukaemia inhibitory factor; OSM, oncostatin M; IFN, interferon; MIP, macrophage inflammatory protein; RANTES, regulated upon activation, normal T cell expressed and secreted; EGF, epidermal growth factor; LPS, lipopolysaccharide; VEGF, vascular endothelial growth factor; MMP, matrix metalloproteinase; hTERT, human telomerase reverse; SLF, steel factor, HCV, hepatitis C virus | | | | |

Table 2 is taken from Johnston PA and Grandis RG 2011 and correlates STAT3 with numerous tumours, confirming the fact that STAT3 is effectively a target of interest for anti-cancer therapies.

**Table 2**

| **Characterisation of tumours with of STAT3 and activity** | **Inauspicious prognosis correlated STAT3** | **with high levels of Abnormality upstream and downstream of the signal of STAT3** | **Models of xenotransplantation responsive to the inhibition of STAT3** |
|---|---|---|---|
| -Leukaemia | -Carcinoma of the kidney cells | -Elevated expression of EGFR | - Squamous-cell carcinoma of the head and neck |
| -Lymphoma | | | |
| -Multiple myeloma | -Colorectal cancer | -Constitutively activated EGFR-RTK | |
| -Breast cancer | -Ovarian carcinoma | | -Glioblastoma |
| -Prostate carcinoma | -Gastric carcinoma | -Overexpression of SFK | -Myeloproliferative neoplasms |
| -Lung cancer | -Intestinal-type gastric adenocarcinoma | | |
| -Lung cancer (not small cell) | | -Hyperactivated JAK | -Carcinoma of the renal cells |
| | -Squamous-cell | -Elevated levels of | |
| -Carcinoma of the renal cells | carcinoma of the cervix | TNF-α/IL-6 | -Breast cancer |
| | | | -Lung adenocarcinoma |
| -Hepatocellular carcinoma | -Osteosarcoma | | -Acute lymphoblastic leukaemia |
| | -Epithelial carcinoma of the ovary | | |
| -Cholangiocarcinoma | | | |
| -Ovarian carcinoma | | | |
| -Pancreatic adenocarcinoma | | | |
| -Melanoma | | | |
| -Squamous-cell carcinoma of the head and neck | | | |

Table 3, taken from TurksonJ and JoveR 2000, indicates numerous tumours associated directly with the anomalous activation of STAT3.

**Table 3**

| Type of tumour | activated STATs | References |
|---|---|---|
| **Breast tumours** | STAT 1, 3 | (Garcia et al., 2000; Watson and Miller, 1995) |
| Tumours | | (J Bromberg and JE Darnell, unpublished results; |
| | | P Chaturvedi and EP Reddy, unpublished results; |
| | | R Garcia, C Muro-Cacho, S Minton, C Cox, N Ku, R |
| | | Falcone, T Bowman and R Jove, unpublished results) |
| cells | STAT 3 | (Garcia et al., 1997; Sartor et al., 1997) |
| **Neck and head tumours** | STAT 1, 3 | (Grandis et al., 1998, 2000a) |
| Cell lines and tumours | | |
| **Malignant melanomas** | STAT 1, 3 | (Florenes et al., 1999; Kirkwood et al., 1999; Pansky et al., 2000) |
| Cell lines and tumours | | |
| **Pituitary tumours** | STAT 1 | (Ray et al., 1998) |
| Cell lines | | |
| **Brain** tumours (primary tumours | | |
| Gliomas | STAT 1, 3 | (Cattaneo et al., 1998) |
| Medulloblastomas | STAT 3 | (Cattaneo et al., 1998) |
| Brain meningiomas | STAT 1,3,5 | (Magrassi et al., 1999; Schrell et al., 1998) |
| **Multiple myelomas** | STAT 1, | (Catlett-Falcone et al., 1999b) |
| Cell lines and tumours | | |
| **Lymphomas** (cell lines and tumours) | | |
| Large T-cell anaplastic lymphoma | STAT 3, 5 | (Zhang et al., 1996c) |
| Sezary syndrome | STAT 3, 5 | (Zhang et al., 1996c) |
| EBV-related/Burkitt's HSV lymphoma | STAT 3 | (Weber-Nordt et al., 1996) |
| Saimiri-dependent HSV (T-cell) | STAT 3 | (Lund et al., 1997b, 1999) |
| T-cell cutaneous lymphoma | STAT 3 | (Sun et al., 1998) |
| LSTRA T-cell lymphoma (mouse) | STAT 5 | (Yu et al., 1997) |
| Mycosis fungoides | STAT 3 | (Nielsen et al., 1997) |
| **Leukaemia** (tumours and cell lines) | | |
| HTLV-I dependents | STAT 3, 5 | (Migone et al., 1995; Takemoto et al., 1997) |
| Chromic lymphocytic leukaemia (CLL) | STAT 1, 3 | (Frank et al., 1997) |
| Acute myeloid leukaemia (AML) | STAT 1, 3, 5 | (Chai et al., 1997; Gouilleux-Gruart et al., 1996; Weber- Nordt et al., 1996) |
| Megakaryocytic leukaemia | STAT 1, 3, 5 | (Liu et al., 1999) |
| Large granular lymphocytic leukaemia (LGL) | STAT 3 | (Epling-Bumette et al., 2000) |
| **OTHER TUMOURS** | | L Mora, R Garcia, J Seigne, T Bowman, M Huang, G Niu, J Pow-Sang, J Diaz, C Muro-Cacho, D Coppola, T Yeatman, J Cheng, S Nicosia, S Shivers, T Landowski, D Reintgen, W Dalton, H Yu and R Jove, unpublished results |
| (tumours and cell lines) | | |
| Prostate | STAT 3 | |
| Renal cell carcinoma | STAT 3 | |
| Ovarian carcinoma | STAT 3 | |
| Melanoma | STAT 3 | |

In particular, in relation to STAT3, the following has been demonstrated in numerous publications:
1) STAT3 is often constitutively active (phosphorylated) in many human cancer cells, such as multiple myeloma, lymphoma, leukaemia, lung cancer, prostate cancer, squamous-cell carcinoma of the head and neck, and other tumour types.
2) STAT3 is activated by growth factors (for example EGF, TGF-α, IL-6, IL-10, IL-23, IL-21, IL-11, HGF), kinase oncogenics (for example Src).
3) STAT3 mediates the expression of proliferation genes (for example c-myc, cyclin D1), of apoptosis suppressor genes (for example Bcl-XL and survivin), of cytokine coding genes, and of genes that promote angiogenesis (for example VEGF), increasing, when activated, cell proliferation and angiogenesis and inhibiting apoptosis.
4) The activation of STAT3 also correlates with phenomena of chemoresistance and radioresistance.
5) The persistent activation of STAT3 increases, in various human cancers, proliferation, survival, angiogenesis and metastasis and inhibits anti-tumour immunity.

It is also known that chronic inflammation in certain organs or at certain sites promotes malignant transformation, and that STAT3 is crucial for the extrinsic and intrinsic pathways of inflammations that lead to cancer, STAT3 being known in fact to guide the malignant characteristics associated with chronic inflammation.

Due to the crucial role of STAT3 in tumourigenesis, the inhibitors of STAT3 have enormous potential in the prevention and in the treatment of cancer. Perhaps one of the best-known inhibitors of the activation of STAT3 is AG490, which inhibits the activation of JAK2. Other inhibitors of STAT3 include small peptides, oligonucleotides, and small molecules. Some authors have identified peptides that block the phosphorylation/activation of STAT3, this being a mechanism that mediates the binding to the DNA and the activity of gene regulation, and cell transformation. Various small molecules that block STAT3 include PGJ2, complexes of platinum, ethanol, sodium salicylate, retinoic acid, atiprimod, PS-341 and statins. Many polyphenol plants have been identified for their ability to suppress the activation of STAT3. These include curcumin, resveratrol, cucurbitacin, piceatannol, parthenolide, flazopiridol, magnolol, and epigallocatechin-3-gallate. The way in which these molecules succeed in suppressing the activation of STAT3 is not entirely clear. For example, curcumin has demonstrated the effect of inhibition of JAK2, Src, Erb2 and EGFR, which are all involved in the activation of STAT3, also downregulating the expression of Bcl-xL, cyclin D1, VEGF, and TNF, of which the expression is regulated by STAT3 (Aggarwal B. B. et al. Ann. N.Y. Acad. Sci. 1091; 151-69: 2006).

There are thus various strategies and various mechanisms that make it possible to intervene in the cascade of signalling of STAT3: inhibiting the phosphorylation/activation of STAT3, inhibiting the intermolecular interactions that involve STAT3, inhibiting the nuclear import/export of STAT3, inhibiting the transcription mediated by STAT3. Apart from the chemotherapeutic agents already mentioned that inhibit STAT3, there are also others (cetuximab, gefitinib, erlotinib, etc.), for which different effects have been reported: a modest efficacy, the development of resistances, myelosuppression, toxicity at gastro-intestinal level, and various adverse events including cardiovascular toxicity (see Table 4).

Table 4, below, taken from Johnston PA and Grandis RG 2011, reports strategies and results for the therapeutic intervention of the signal of STAT3.

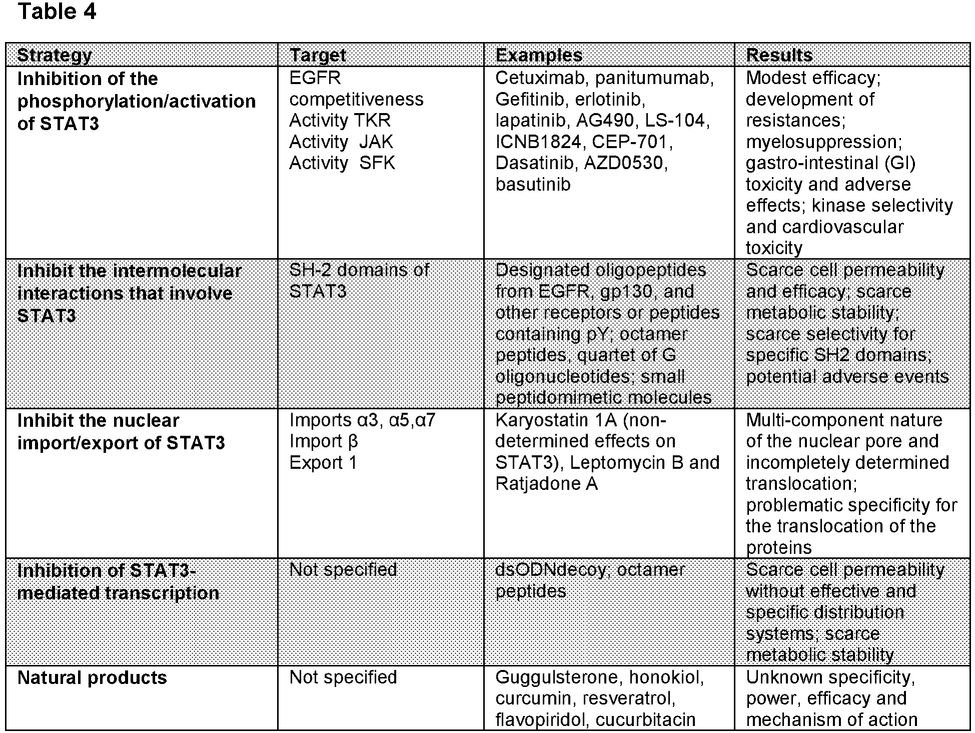

Therapies that are targeted therapies by means of compounds that inhibit a specific target molecule in a more specific manner, in sub-populations of cells directly involved in tumour progression, represent a new perspective in the treatment of cancer. The molecules that control cell proliferation and death, such as receptor tyrosine kinases (RTKs) for growth factor are among the best objectives of this type of therapeutic approach. The era of targeted therapy started with the approval of trastuzumab, a monoclonal antibody against HER2, for the treatment of metastatic mammary carcinoma and imatibin, an inhibitor of BCR-ABL, in chronic myeloid leukaemia. In spite of the initial enthusiasm for the efficacy of these treatments, the doctors had to immediately confront the problem of relapse, since those suffering from cancer almost always developed a resistance to the drugs, often due to the activation of alternative pathways. Since the tumour is characterised by more mechanisms and more gene targets, which are frequently deregulated, it would be advantageous to adopt a combination therapy, as is standard in the treatment of cancer, since this results in a rational strategy for increasing the response and the tolerability and for decreasing resistance. There is currently a rise in interest for the combination of anti-tumour drugs that aim to maximise efficacy, minimising the systemic toxicity by means of the use of lower drug doses.

Thus, pharmacologically safe and effective therapeutic agents, such as molecules of natural origin, which can block constitutive or inducible activation of STAT3, have a potential efficacy in the treatment of cancer, given that more and more tests are concluding that the inhibition of the phosphorylation of STAT3 by means of a pharmacological blocking of the molecules upstream, including Src and JAK, can reduce the formation of tumours, also leading to the possibility of reduction of the necessary dosage of chemotherapeutic drug.

In addition, since the activation of STAT3 also correlates with the resistance to chemotherapy and radiotherapy, inhibitors of such activation are also of great interest for limiting such resistance and optimising the effect of chemotherapy and of radiotherapy.

### SUMMARY OF THE INVENTION

The authors of the present invention have demonstrated that extracts of Filipendula *(Filipendula vulgaris*) are able to selectively modulate, essentially inhibit, the phosphorylation of the protein STAT3, consequently preventing the subsequent action within the cell as transcription factor. As will be seen in the experimental part of the application, the authors of the invention have demonstrated, in numerous experiments and on various cell lines, that the extracts described here are effective inhibitors of the activation (phosphorylation) of STAT3 and consequently
- demonstrate effective cytotoxic action on tumour cell lines,
- are able to inhibit the regeneration of tumour cells, thus acting as cytostatics,
- induce apoptosis in tumour cells
- have additive and also synergistic effects with numerous chemotherapeutic agents, thus resulting in a reduction of the vitality of the tumour cells compared with those treated with the chemotherapeutic agent alone or with the extract alone.

From the viewpoint of the effect of such extracts on the STAT3 factor, the authors of the present invention have also demonstrated by way of experiment that the extracts of *Filipendula vulgaris* described here are able to prevent the binding of STAT3 to the DNA and thus to prevent the alteration of the expression of the genes normally activated by phosphorylated STAT3.

In other words, said extract has proven to be capable of modulating, essentially inhibiting, the protein STAT3 in its phosphorylated form, preventing the successive action of said protein within the cell as transcription factor. In particular, the inventors of the present disclosure have demonstrated that an extract of *Filipendula Vulgaris* is able to inhibit the constitutive or anomalous activation of STAT3 and to induce the reactivation of apoptosis in cultures of MPM tumour cells. In addition, the authors of the present invention have also demonstrated that, in experiments on cultures of tumour cells, the extract of *Filipendula Vulgaris* inhibits woundhealing, in fact preventing the invasivity of the tumour cells. In addition, the authors of the present invention have also demonstrated with experiments of engraftment of tumour cells in mice that the extract of the present invention exerts *in vivo* an anti-tumour effect.

A first subject of the present invention is therefore an extract of *Filipendula Vulgaris* for use in the prevention and/or in the treatment of an inflammatory and/or pre-tumour and/or tumour pathological condition characterised by a constitutive or anomalous activation of the STAT3 transcription factor.

A second subject of the present invention is an extract of *Filipendula vulgaris* for use in the prevention and/or in the treatment of an inflammatory and/or pre-tumour and/or tumour pathological condition characterised by a constitutive or anomalous activation of the STAT3 transcription factor in association with one or more chemotherapeutic agents. A third subject of the present disclosure is a composition comprising an extract of *Filipendula vulgaris* and a carrier and/or diluent and/or excipient for use in the prevention and/or in the treatment of an inflammatory and/or pre-tumour and/or tumour pathological condition characterised by a constitutive or anomalous activation of the STAT3 transcription factor.

In one embodiment the composition also comprises one or more components with anti-apoptotic activity.

A fourth subject of the present invention is a composition comprising an extract of *Filipendula vulgaris* in association with one or more components with anti-tumour and/or anti-inflammatory activity and a carrier and/or diluent and/or excipients for use in the prevention and/or in the treatment of an inflammatory and/or pre-tumour and/or tumour pathological condition characterised by a constitutive or anomalous activation of STAT3 transcription factor.

A fifth object of the present invention is a kit for concomitant or sequential administration of an extract of *Filipendula vulgaris* and one or more chemotherapeutic agents comprising one or more aliquots of an extract of *Filipendula vulgaris* or of a composition comprising an extract of *Filipendula vulgaris* and one or more separate aliquots of one or more compositions comprising a chemotherapeutic agent for use in the prevention and/or in the treatment of an inflammatory and/or pre-tumour and/or tumour pathological condition characterised by a constitutive or anomalous activation of STAT3 transcription factor in association with a chemotherapeutic agent.

A sixth subject of the invention is a therapeutic method for the prevention and/or in the treatment of an inflammatory and/or pre-tumour and/or tumour pathological condition characterised by a constitutive or anomalous activation of the STAT3 transcription factor comprising the step of administering to an individual who needs it a therapeutically active quantity of extract of *Filipendula vulgaris,* of pharmaceutical composition comprising extract of *Filipendula vulgaris,* optionally in association with one or more components having anti-tumour and/or anti-tumour activity.

### DETAILED DESCRIPTION OF THE FIGURES

Note: In the present figures, the extract of *Filipendula vulgaris* used is often indicated by the abbreviation ABO-2.
**Figure 1****:** Inhibition of the phosphorylation of STAT3, p-STAT3 (Y705)
Figure 1A Western Blot analyses of cell lysates obtained from MSTO211H treated with 100 µg/ml of *Filipendula vulgaris* extract for 24 hours. Quantification was performed compared with a control of Actin.
Figure 1B Br chart of the data obtained with Western Blot on MSTO211H cells. p-STAT3 (phosphorylated STAT3) is shown in black, STAT3 is shown in grey.
   The figure shows that the extract inhibits the formation of p-STAT3 compared with the control.
**Figure 2****:** Clonogenic assay (see the experimental section for the conditions) on cell lines of human mesothelioma with various doses of extract of *Filipendula vulgaris*
   graph 2a. assay performed on human mesothelioma cell line MSTO211H
   graph 2b : assay performed on human mesothelioma cell line MPP89
**Figure 3****:** The extract of the invention influences the ability of 3 different inflammatory tumour lines (MDA-MB-231, DU145E, HCT116) to form colonies, in a dose-dependent manner, independently of the isotypes thereof.
   graph 3a. assay performed on breast tumour cell line MDA-MB-231
   graph 3b. assay performed on prostate tumour cell line DU145
   graph 3c. assay performed on colon tumour cell line HCT116
**Figure 4****:** Assay of cell vitality using ATPlite assay (see the experimental section for the conditions) on mesothelioma cell lines (MSTO211H, MPP-89,NCI-H28). The assay shows that cell vitality is inhibited by the extract of *Filipendula vulgaris* of the invention, in a dose-dependent manner in various mesothelioma cell lines.
**Figure 5****:** comparison of the three vitality curves of Figure 4 compared with (Figure 5a MSTO211H, Figure 5b MMP-89, Figure 5c NCI-H28) the proliferation curve obtained treating normal mesothelial cells (HMC) with extract of *Filipendula vulgaris.* The malignant mesothelioma cell lines (MPMs) clearly show the anti-proliferative effect of the extract of *Filipendula vulgaris* compared with the HMCs.
**Figure 6****:** Assay of cell vitality (ATPlite assay) following treatment with *Filipendula vulgaris* extract in association with Pemetrexed (PMTX) on mesothelioma cell lines MPM MSTO211H.
   In Figure 6a the effect of various concentrations of *Filipendula vulgaris* extract on the cells, in Figure 6b the effect of various concentrations of pemetrexed on the cells, and in Figure 6c the association between increasing doses of pemetrexed and a minimum dose of 6µg/ml of *Filipendula vulgaris* extract are reported.
   The treatment with PMTX is cytotoxic for the MPM cells and highly toxic for the non-tumour cells. The co-treatment of the cells with the extract of the invention + PMTX had a significant effect on cell vitality in MPM cell lines.
**Figure 7****:** Assay of cell vitality (ATPlite assay) following treatment with *Filipendula vulgaris* extract in association with Pemetrexed (PMTX) on mesothelioma cell lines MPM NCI-H2052.
   In Figure 7a the effect of various concentrations of *Filipendula vulgaris* extract on the cells, in Figure 7b the effect of various concentrations of pemetrexed on the cells, and in Figure 7c the association between increasing doses of pemetrexed and a minimum dose of 6µg/ml of *Filipendula vulgaris* extract are reported.
   The treatment with PMTX is cytotoxic for the MPM cells and highly toxic for the non-tumour cells. The co-treatment of the cells with the extract of the invention + PMTX had a significant effect on cell vitality in MPM cell lines.
**Figure 8****:** Assay of cell vitality (ATPlite assay) following treatment with *Filipendula vulgaris* extract in association with Pemetrexed (PMTX) on mesothelial cells (HMC). As can be seen in the preceding Figures 6 and 7, the treatment with PMTX is cytotoxic for the MPM cells and highly toxic for the non-tumour cells.
   In Figure 8a the effect of various concentrations of *Filipendula vulgaris* extract on the cells, in Figure 8b the effect of various concentrations of pemetrexed on the cells and in Figure 8c the association between increasing doses of pemetrexed and a minimum dose of 6µg/ml of *Filipendula vulgaris* extract are reported.
   The co-treatment of the cells with the extract of the invention + PMTX had a significant effect on cell vitality in MPM cell lines, whilst reducing the mortality caused by Pemetrexed in the untransformed cells (HCM). Consequently, it is clear that the extract of *Filipendula vulgaris* of the invention makes only the tumour cells sensitive to pemetrexed.

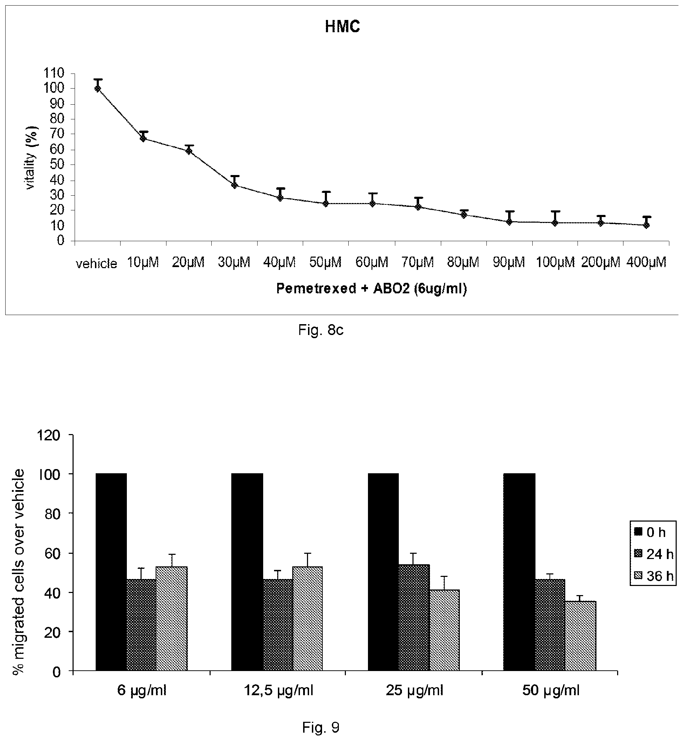

**Figure 9****:** woundhealing assays on human mesothelioma cell line MSTO221H (see experimental section for the conditions).
   The figure shows bar charts concerning the efficacy in closing the wound (quantification of the number of cells in %, migrated compared with the carrier) treated with the extract of the invention and with carrier at the times indicated. The figure shows that the number of migrated cells sensibly reduces in the presence of the extract of *Filipendula vulgaris.*

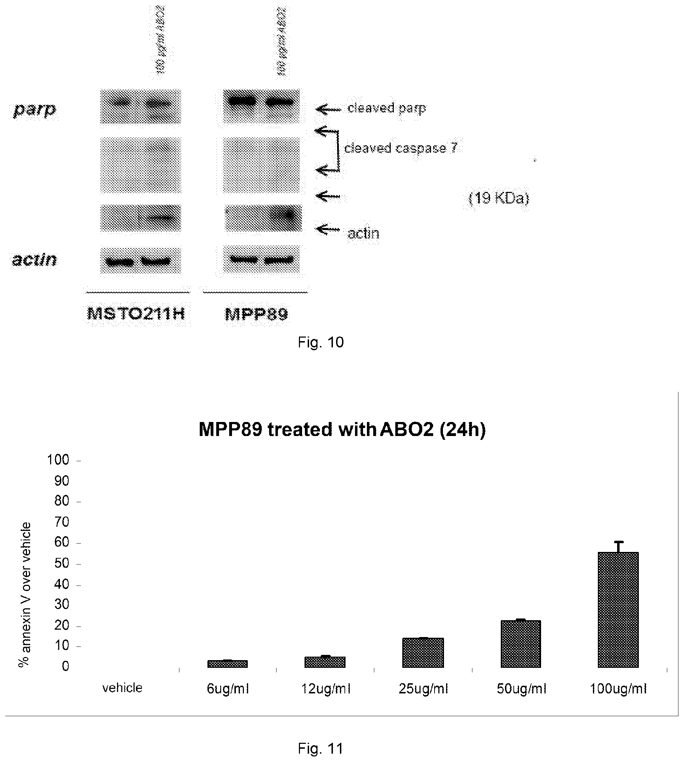

**Figure 10****:** Assays to assess the induction of apoptosis (Western method). The extract of the invention at the dose of 100 µg/ml induces apoptosis as demonstrated by the rise in the levels of some apoptotic markers as the cleaved form of PARP and of caspases 3 and 7 in the cell line MSTO211H.
**Figure 11****:** Assay to assess the induction of apoptosis by means of measurement of the level of annexin V. The extract of the invention induces apoptosis in the cell line MPP89, as determined by the coloration of annexin V, in a time-dependent and dose-dependent manner.

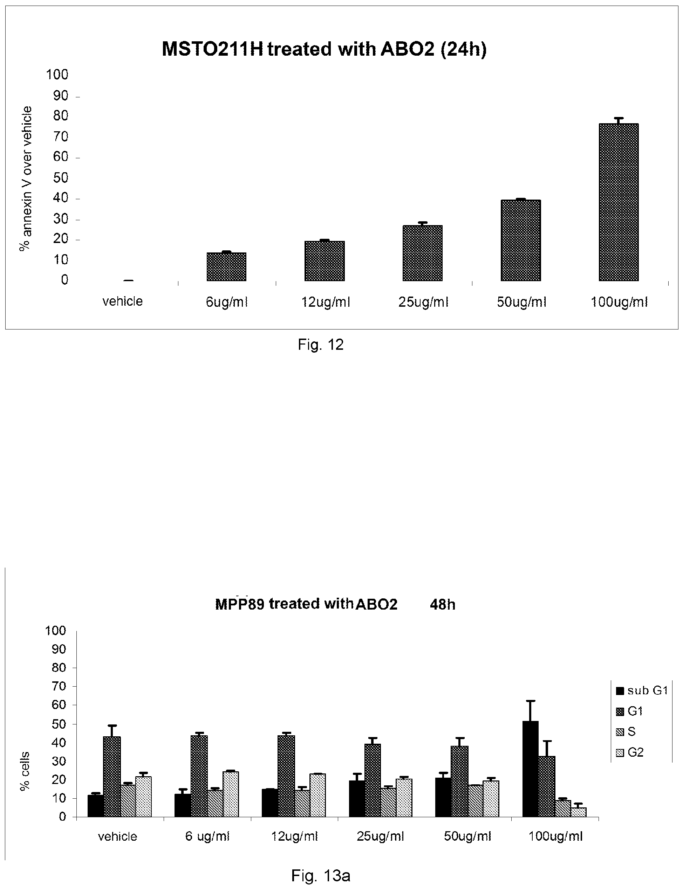

**Figure 12****:** Assay to assess the induction of apoptosis by means of measurement of the level of annexin V. The extract of the invention induces apoptosis in the cell line MSTO211H, as determined by the coloration of annexin V, in a time-dependent and dose-dependent manner.

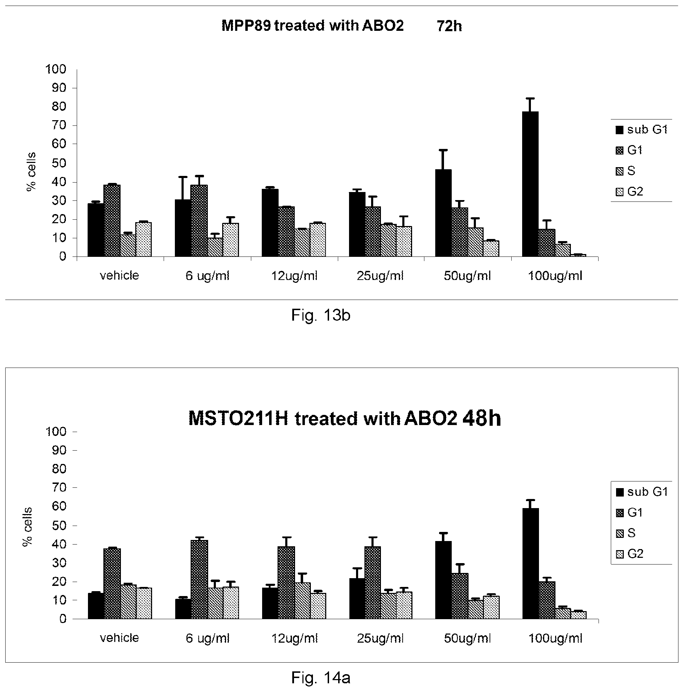

**Figure 13****:** Analyses of the cell cycle after treatment with ABO2 for 48 hours and 72 hours (Figures 13a and 13b) on human mesothelioma cell lines MPP89.
   The graph demonstrates that the extract of the invention blocks in a time-dependent manner the cells in sub G1 phase, actually preventing the progression of cell replication in the assayed mesothelioma cells.
   After 72 h of treatment, Figure 13b, almost no cell reaches G2 phase which precedes mitosis.

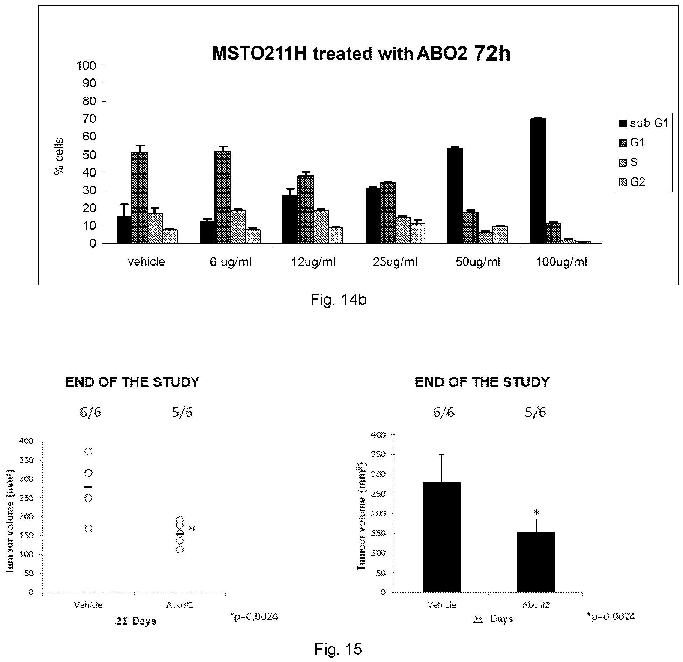

**Figure 14****:** Analyses of the cell cycle after treatment with ABO2 for 48 h and 72 h (Figures 14a and 14b) on mesothelioma cell lines MSTO211H.
   The graph demonstrates that the extract of the invention blocks in a time-dependent manner the cells in sub G1 phase, actually preventing the progression of cell replication in the assayed mesothelioma cells.
   After 72 h of treatment, Figure 14b, almost no cell reaches G2 phase which precedes mitosis.
**Figure 15****:** Effect of the Filipendula extract on the engraftment of MPM cell lines
   The MSTO211H cells were pre-treated with *Filipendula vulgaris* for 24 hours. Then, they were inoculated in nude CD1 mice. The pre-treatment with the *Filipendula vulgaris* extract influenced the engraftment of the tumour and induced a statistically significant difference (p=0.0024) in the volume of the tumour.

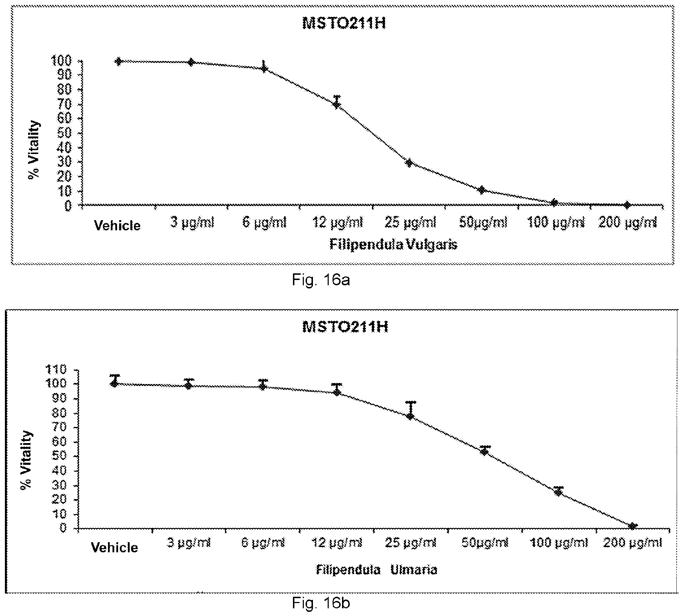

**Figure 16****:** Assay of cell vitality with ATPlite assay (see experimental section for the conditions) on mesothelioma cell lines MSTO211H treated with an extract of *Filipendula vulgaris* (Figure 16a) and an extract of *Filipendula ulmaria* (dropwort). The assay shows that cell vitality is inhibited by the extract of *Filipendula vulgaris* of the invention, in a dose-dependent manner in various mesothelioma cell lines.

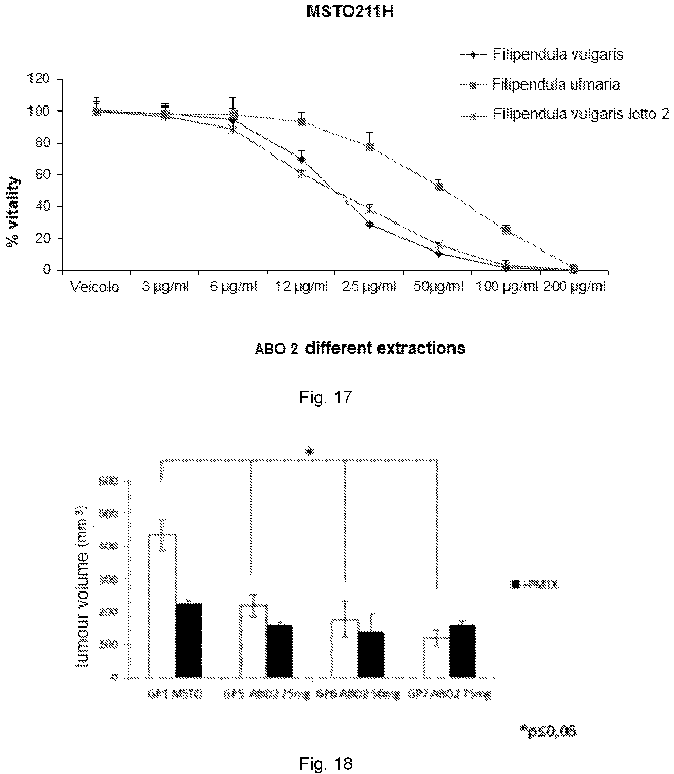

**Figure 17****:** comparison of the two vitality curves obtained by treating mesothelioma cell lines MSTO211H with *Filipendula vulgaris* extract with respect to the proliferation curve obtained by treating the same cell lines with *Filipendula ulmaria* (dropwort) extract. Malignant mesothelioma cell lines (MPMs) are affected to a greater extent by the anti-proliferative effect of the *Filipendula vulgaris* extract compared to the *Filipendula ulmaria* extract, as is clear from the figure.
**Figure 18****:** Effect of *Filipendula vulgaris* extract administered together with Pemetrexed *in vivo* on mice.
   Groups were schematized as reported in Table 5 of Example 13.

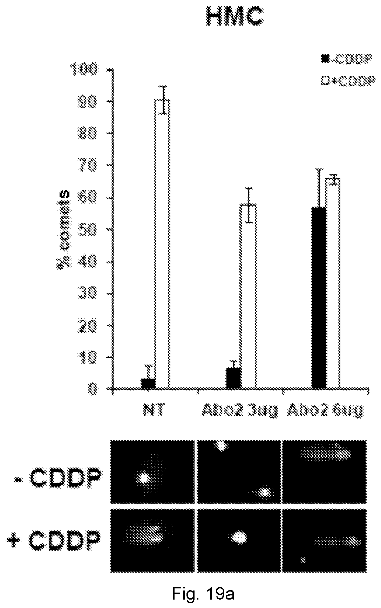

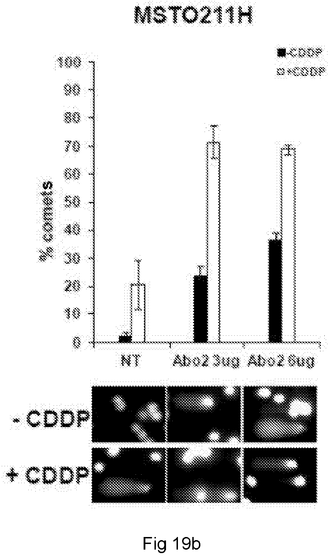

**Figure 19****:** As shown by comet assay, the treatment with ABO2 did not induce DNA damage in untransformed normal mesothelial cells (HMC).

Conversely, ABO2 induced DNA damage in MSTO211H cells.

The treatment with CDDP (cis-diamminedichloroplatinum) induces DNA fragmentation in both cell lines.

The treatment with ABO2 protects normal cells from CCDP-induced DNA fragmentation, whilst in MSTO211H cells it exhibits a synergistic effect with CDDP in inducing DNA fragmentation.

### DETAILED DESCRIPTION OF THE INVENTION

The present application thus relates to a use of the extract of *Filipendula vulgaris* in the prevention and/or in the treatment of pathologies in which a constitutive or anomalous activation of the STAT3 factor is present. As indicated before, the anomalous or constitutive activation would appear to consist in an anomalous or constitutive phosphorylation of this factor with resultant inflammatory and/or tumourigenic effects both in the blood and in tissues.

In the following description, in the claims and in the drawings, the term "STAT3" denotes the transduction factor of the signal and activation of STAT3 transcription (Signal Transducer and Activator of Transcription 3). Conventionally, where reference is made to the gene, uppercase italicised letters are used, whereas the protein is indicated by non-italicised uppercase letters.

It is already known in the literature that inflammation and tumours are closely linked by oncogenic and environmental pathways, and the phosphorylation of the STAT3 factor (Signal Transducer and Activator of Transcription 3) causes activation thereof and the displacement into the nucleus where it acts as an activator of the transcription of numerous cytokines, chemokines, and other mediators associated with inflammation, thus promoting cancer.

Inhibitors of the activation of STAT3 are therefore factors that have a preventative and/or curative effect towards all those pathologies in which constitutive activation of the STAT3 factor is present. The present invention discloses for the first time the inhibitory action specific for STAT3 of extracts of *Filipendula vulgaris.*

*Filipendula vulgaris* is also known as *Spiraea vulgaris, Spiraea filipendula* or *Filipendula hexapetala.* For the purposes of the present invention, except when specifically indicated, the term Filipendula vulgaris means solely *Filipendula vulgaris* or a synonym thereof as defined above, and not Filipendula ulmaria (dropwort).

Filipendula vulgaris is a herbaceous perennial plant, up to 70 cm tall, with erect, streaked, glabrate and scarcely branched stems, whose leaves have stipules which are pennato-septate or with a double coarse crenation, and the hermaphroditic flowers are cream white or pink, generally have 6 petals, and are reunited in corimbous apical tops.

This plant is commonly known for its antirheumatic, depurative, diuretic and astringent properties.

For the purposes of the implementation of the present invention, the extract may be an extract of *Filipendula vulgaris* flowering top (leafy inflorescence), by 'flowering top' meaning the aerial portion of the plant harvested at a 10-15 cm height from the ground, characterised by stem, leaves and flowers (leafy inflorescence, inflorescence). Harvesting time is from May to October, depending on seasonal climate patterns. The extract can be made both from the fresh plant and the dried one. In this latter case, drying is carried out in a ventilated furnace, at a temperature of about 35-45°C.

The extract according to the invention could be a fluid extract, a soft extract, or an extract lyophilised or dried by means of known drying techniques. The extract can be obtained by means of extraction with the following solvents: water, ethanol, methanol, acetone or isopropanol, in each case in pure form or in a mixture with one another. The alcohol could be methanol, ethanol, isopropanol and is preferably ethanol. The ethanol can be used in pure form or in mixture with water at the following percentages: 96%, 90%, 85%, 80%, 75%, 70%, 65%, 60%, 55%, 50%, 45%, 40%, 35%, 30%, 25%, 20%, 15%, 10%, 5%, 1%. In a non-limiting embodiment of the invention, the solvent used for the extraction could be a mixture formed by ethyl alcohol and water in a proportion of 50:50. The fluid extract could be prepared by means of hydroalcoholic extraction by percolation/digestion of the above-indicated parts of *Filipendula vulgaris* in relation to drug/solvent from 1:2 to 1:100 and preferably in a ratio of 1:10. The duration of the extraction is a duration commonly used by a person skilled in the art and could be, for example, from a minimum of 4 hours to approximately 8 hours. The temperature of extraction is normally controlled and could preferably be, for example, a temperature of approximately 50°C. The evaporation of the alcohol from the hydroalcoholic extract and the subsequent drying of the aqueous concentrate could be performed by means of lyophilisation or desiccation to provide the lyophilised extract or dry extract.

The preparation of such extracts is commonly known to a person skilled in the art and does not need to be described in particular detail in the present disclosure. For the purposes of implementing the present invention, it is possible to use any extract among those indicated above, prepared in accordance with conventional techniques.

In particular, for the purposes of the present invention, the extract could also be a fraction of the extracts as described here.

In this case, a standard procedure comprises the evaporation of the alcohol present in the alcoholic extract at 50 alcoholic degrees, the removal of the substances insoluble in alcohol by means of centrifugation at 4000 rpm for 1-5 minutes, the introduction of the aqueous solution (aqueous concentrate), resulting in a chromatographic column containing the adsorption resin.

The aforementioned aqueous concentrate can in turn be obtained by suspending the dried or lyophilised *Filipendula vulgaris* extract in water at a ratio of 1:10 w/w.

The feed fluid of the resin must have suspended solids indicatively comprised between 0.2 at 1° Bix, the feed range is between 1 and 4BV/hour and preferably 2 BV/hour. The corresponding aqueous eluate is collected and subjected to drying by means of lyophilisation or desiccation, the substances adsorbed into the resin being eluated using 96% ethyl alcohol or methanol or acetone, preferably 96% ethyl alcohol. In this last case, the alcohol eluate is subjected to desiccation or to lyophilisation after having added in this last case water at a ratio of 1:1 v/v with alcohol eluate and ethyl alcohol evaporate.

In accordance with the present invention, the extract of *Filipendula vulgaris* as defined above could be used for the prevention and/or the treatment of pathologies characterised by a constitutive or anomalous activation of the STAT3 transcription factor.

Such diseases can be, for example and as noted in the literature, diseases of the inflammatory and/or pre-tumour and/or tumour type..

For the purposes of the present invention, the pathological states characterised by a constitutive or anomalous activation of the STAT3 transcription factor can be caused by viral infections (as noted in the literature), including infections by *H pylori,* infections by the Hepatitis B virus, infections by HPV (human papilloma virus), infections by the Epstein-Barr virus (as reported in Yu et al 2009).

As already mentioned, the term "STAT3" thus denotes the human transcription factor "Signal transducer and activator of transcription 3", coded in humans by the STAT3 gene.

The invention concerns pathological states in humans defined in detail in the present description (for example below) in which this gene is activated constitutively or anomalously in any case.

The pre-tumour pathological states in which a constitutive or anomalous activation of STAT3 is present can be either pathological states following the ablation of a tumour, and thus pre-tumour in the sense that the tumour could reform, or pathological states in which there is a transfer from inflammation to the acquisition of malignant characteristics on the part of the cell, as reported in the literature.

In accordance with the present invention, the tumour pathological states can be any tumours characterised by a constitutive or anomalous activation of STAT3 reported in the prior art, such as:
prostate cancer, multiple myeloma, leukaemia, lymphoma, melanoma, carcinoma of the ovaries, carcinoma of the renal cells, pancreatic adenocarcinoma, lung cancer, brain tumour, erythroleukaemia, squamous-cell carcinoma of the head and neck, colon cancer, mesothelioma

More specifically, said brain tumour could be, for example, a glioma, a brain meningioma, a medulloblastoma, said lymphoma could be Sezary syndrome, EBV-associated Burkitt lymphoma, Saimiri HSV-dependent lymphoma, cutaneous T-cell related lymphoma; said leukaemia may be HTLV-I-dependent leukaemia, chronic lymphocytic leukaemia (CLL), acute myelogenous leukaemia (AML), megakaryocytic leukaemia, large granular lymphocytic leukaemia (LGL).

In accordance with a non-limiting example of the present invention, the extract of *Filipendula vulgaris* as defined above can be used for the prevention and/or the treatment of any one of the pathological states characterised by a constitutive or anomalous activation of STAT3 listed in Table 1 above.

The terms "constitutive activation" or "anomalous activation" according to the present invention are to be understood within the sense of the meaning attributed to such terms in the literature relating to STAT3 (for example as listed in the bibliography), or a persistent activation of this factor, usually absent in healthy cells.

Given the specificity in the inhibition of the activation and of the activity of STAT3 shown by the extract of the invention, the extract of the present invention can thus be used in the treatment of tumour pathologies resistant to treatment with chemotherapeutic agents that do not inhibit STAT3. A non-limiting example of chemotherapeutic agents that do not inhibit STAT3 is represented by the chemotherapeutic drugs used for mesothelioma, which is a tumour with pSTAT3 constitutively activated and highly chemo-resistant. Examples of the agents commonly used include pemetrexed, which is an inhibitor of thymidylate synthase; methotrexate, which is a competitive and reversible inhibitor of dihydrofolate reductase; gemcitabine, which inhibits the synthesis of DNA as a false substrate in the biosynthetic pathways of the pyrimidine nucleotides; vinorelbine, which is an antimitotic drug that binds to the monomers of tubulin, inhibiting the formation of microtubules; cisplatinum, which is an agent able to interfere with all the phases of the cell cycle binding to the DNA by means of the formation of interfilament and intrafilament cross-links in the DNA.

The experimental data presented below and in the figures, obtained on tumour cell lines in which the constitutive activation of STAT3 is known, also show that the extract of *Filipendula vulgaris* according to the invention cay be associated advantageously with one or more anti-tumour drugs, thus increasing, also synergically, the anti-tumour efficacy of the drugs themselves.

Thus, in accordance with an embodiment of the present invention, the extract of *Filipendula vulgaris* as described here can be used in the prevention and/or in the treatment of an inflammatory and/or pre-tumour and/or tumour pathological condition characterised by a constitutive or anomalous activation of the STAT3 transcription factor, in association with one or more compounds having anti-tumour activity and/or one or more compounds having anti-inflammatory action.

In accordance with an embodiment, the compound having anti-tumour activity can be a chemotherapeutic agent and can be selected in the group comprising cisplatinum, doxorubicin, pemetrexed, methotrexate, vinorelbine, gemcitabine and taxol.

The present invention thus comprises the use of extract of *Filipendula vulgaris* as defined here in association with one or more chemotherapeutic agents for the prevention and/or the treatment of tumour or pre-tumour pathological states characterised by a constitutive or anomalous activation of STAT3.

The association with one or more chemotherapeutic agents may be a concomitant or sequential association, or the extract and the chemotherapeutic agents can be administered at the same time (in a single administration or in separate administrations) or over a period of time of a few minutes, or can be administered sequentially or at different times, separated from one another by more than a few minutes, over the course of the day or the period of therapeutic treatment.

The administration regime will be determined by the treating doctor in accordance with the sex, the age, the state of disease, the weight and the history of the patient.

Both alone and in association, as described above, the treatment can be preventative, for example in known cases of infection so as to have possible tumourigenic effects such as those indicated above, or in the case of ablation of tumours so as to prevent said tumours from reforming.

The extract according to the present invention can be formulated in compositions that can be used for the same objectives as described above.

The present invention therefore further relates to a composition comprising, as active ingredient, an extract of *Filipendula vulgaris* and a carrier and/or diluent and/or excipient for use in the prevention and/or in the treatment of an inflammatory and/or pre-tumour and/or tumour pathological condition characterised by a constitutive or anomalous activation of the STAT3 transcription factor.

As indicated above, the extract of *Filipendula vulgaris* for the purposes of the present invention can be a plant extract belonging to the *Filipendula vulgaris* genus in accordance with the examples and the definitions provided above.

The composition may comprise, as active ingredient, an extract as defined above in the form of lyophilised extract, a dry extract and/or a fluid extract. As already indicated, the extract can be obtained by extraction of the flowering tops (leafy inflorescences) of *Filipendula vulgaris,* whether fresh or dried. The extraction can be performed by means of percolation-digestion, keeping the temperature controlled at 50°C, the solvent of extraction is represented for example by water, 96% ethanol, methanol, acetone, isopropanol, either as such or in mixture. The fluid extract obtained can then be subjected to evaporation, and subsequent lyophilisation or desiccation provides the lyophilised extract or the dry extract. In accordance with the present invention, the composition as defined above can be used for the prevention and/or the treatment of pathologies characterised by a constitutive or anomalous activation of the STAT3 transcription factor.

Such diseases can be, for example and as noted in the literature, inflammatory and/or pre-tumour and/or tumour diseases. The definition of the various pathological states for which the composition of the invention can be used is the same as that specified above in relation to the therapeutic use of the extract of the invention.

For the purposes of the present invention, the composition can treat pathological states characterised by a constitutive or anomalous activation of the STAT3 transcription factor as already defined above, tumour pathological states as already defined above characterised by a constitutive or anomalous activation of STAT3, and pre-tumour pathological states in which a constitutive or anomalous activation of STAT3 is present as already illustrated beforehand within the scope of the present description.

In accordance with a non-limiting example of the present invention, the composition as defined here can be used for the prevention and/or the treatment of any one of the pathological states characterised by a constitutive or anomalous activation of STAT3 listed in Table 1 above.

In addition, in accordance with a further embodiment, the composition of the invention as described here may further comprise, as active ingredients, one or more anti-tumour agents and/or one or more anti-inflammatory agents.

The present invention therefore further relates to a composition comprising, as active ingredients, extract of *Filipendula vulgaris* and one or more compounds having anti-tumour and/or anti-inflammatory activity for use in the prevention and/or in the treatment of an inflammatory and/or pre-tumour and/or tumour pathological condition characterised by a constitutive or anomalous activation of the STAT3 transcription factor.

In accordance with an embodiment, such compounds having anti-tumour activity may be chemotherapeutic agents selected, for example, in the group comprising cisplatinum, doxorubicin, pemetrexed, methotrexate, vinorelbine, gemcitabine and taxol.

The composition of the invention can be formulated in unit doses or in a dosable manner by the treating doctor for the purpose of also enabling therapies adapted to the individual needs of each patient.

The present invention thus includes the use of compositions comprising an extract of *Filipendula vulgaris* as defined here, optionally in association with one or more further active ingredients having anti-tumour activity and/or one or more further active ingredients having anti-inflammatory activity for the prevention and/or the treatment of tumour and/or inflammatory and/or pre-tumour pathological states characterised by a constitutive or anomalous activation of STAT3.

Such further active ingredients may be, for example, chemotherapeutic compounds, and the pathological states may be pre-tumour or tumour pathological states.

The association with the one or more chemotherapeutic agents may be a concomitant or sequential association, or the extract and the chemotherapeutic agents can be administered at the same time (in a single administration or in separate administrations) or over a period of time of a few minutes, or can be administered sequentially or at different times, separated from one another by more than a few minutes, over the course of the day or the period of therapeutic treatment.

The administration regime will be determined by the treating doctor in accordance with the sex, the age, the state of disease, the weight and the history of the patient.

Both alone and in association, as described above, the treatment can be preventative, for example in known cases of infection so as to have possible tumourigenic effects such as those indicated above, or in the case of ablation of tumours so as to prevent said tumours from reforming.

The composition with one or more active ingredients as described above (extract of *Filipendula vulgaris,* optionally in association with one or more anti-tumour agents and/or one or more anti-inflammatory agents) may obviously comprise one or more excipients or adjuvants technically used in common pharmaceutical or cosmetic practice or in the food industry. The excipients used may belong to the category of diluents, solubilisers, disintegrators, binders, lubricants, surfactants, slip agents and anti-adherents.

If necessary, the composition may also contain flavourings, colorants and preservatives used commonly in the pharmaceutical, cosmetic and food industries.

The composition according to the invention can be prepared in accordance with techniques known to a person skilled in the art and using an extract of *Filipendula vulgaris* as defined above, optionally one or more anti-tumour agents, and one or more excipients belonging to the above-mentioned categories.

The compositions can be in any formulation considered suitable by a person skilled in the art preparing formulations intended for oral administration (for example powders, granulates, capsules in hard or soft gelatine, tablets, syrups, drops, solutions and oral emulsions), inhalation (for example aerosols, liquid and powder sprays), topical administration (gels, ointments, emulsions, pastes, foams, anhydrous solid forms for topical application, and patches) and parenterally in accordance with the techniques currently used and known to a person skilled in the art (for example for subcutaneous use, intramuscular use, intravenous use or intradermal use). In all formulations, the use of technological excipients or adjuvants is determined by selecting the substances to be used on the basis of those used commonly in pharmaceutical practice.

In the preparation of formulations based on extract of *Filipendula vulgaris* in association or not with agents having anti-tumour activity, a person skilled in the art could use any of the excipients deemed useful in accordance with the prior art in order to obtain a stable preparation suitable for use in therapy. By way of example, in the category of diluents, it is possible to use diluents in solid formulations, such as sugars, polyalcohols (for example lactose, manitol, sorbitol), celluloses, salts of inorganic acids (for example dibasic calcium phosphate), salts of organic acids including citrates, carbonate and bicarbonate titrates in the form of salts of sodium, potassium and calcium, or diluents in liquid forms, such as water, edible oils for oral use (sunflower oil, olive oil, corn oil, sweet almond oil, nut oil) or used in topical formulations (jojoba oil, short-chain, medium-chain or long-chain triglycerides), polyalcohols (glycerine, propylene glycols, hexylene glycol).

In the category of the disintegrators, it is possible to use, for example, natural or modified starches (corn starch, rice starch, potato starch), croscaramellose sodium, glycolate sodium starch, crospovidone; possible binders that can be used include natural products of the rubber type (guar gum, xanthan gum, gum arabic), sucrose and synthesis products, including polyvinyl pyrrolidone and semi-synthetic derivatives of cellulose.

The use of stearic acid and salts thereof, including the salt of magnesium, polymers of ethylene glycol, triglycerides and natural or synthetic waxes as lubricants has proven to be effective.

The surfactants are used to make one or more active ingredients contained in the formulations forming the basis of the invention more soluble or washable with water, these active ingredient acting alone or carried by one or more diluents. For example, sorbitan esters, sorbitan polyoxyethylene esters, sucrose esters and sodium lauryl sulphate can be cited.

The slip agents may be selected for example from colloidal silica, precipitated silica, whereas the anti-adherents that can be used include, for example, talc or starch.

In the preparation of injectable formulations, it is possible to choose those excipients that allow effective solubilisation or dispersion of the active substance(s). By way of example, together with water, other hydrosoluble carriers such as polyalcohols and salts of organic or inorganic acids can be used for the purpose of obtaining pH and osmolarity suitable for the administration by means of injections.

In particular cases, it will be possible to use non-hydrosoluble carriers, such as oils, or substances of synthesis commonly approved for injective use.

A person skilled in the art can prepare all the formulations using the common preparation schemas known to him.

Merely by way of example, a formulation in capsules can be prepared conveniently by grinding beforehand the extract of *Filipendula vulgaris,* mixing in a common mixer the powder obtained together with one or more anti-tumour agents and the excipients selected to prepare the formulation, for example a diluent, a disintegrator, a lubricant and a slip agent selected from those mentioned above or available on the market and approved for oral use.

In the case of a tablet, it could be necessary to granulate some or all of the mixture with a binding agent dissolved beforehand in water or introduced in mixture and using the water as an adjuvant of the process of granulation in accordance with the prior art.

The granulate may be dried, sieved and further mixed with other powders for the purpose of obtaining a mixture suitable for obtaining tablets in accordance with that known to a person skilled in the art.

In the case of parenteral use, the composition may also be provided with the active ingredients in separate containers conveniently miscible in accordance with specific operational requirements.

For the purpose of facilitating the use of the compositions described here, these can be presented in the form of unit doses containing one of the active ingredients described here (extract of *Filipendula vulgaris* and optionally one or more anti-tumour agents and/or one or more anti-inflammatory agents) effective for a preventative and/or therapeutic use of a particular pathological condition characterised by a constitutive or anomalous activation of the STAT3 transcription factor.

The present invention further relates to a kit for the concomitant or sequential administration of an extract of *Filipendula vulgaris* and one or more compounds having anti-tumour activity and/or one or more compounds having anti-inflammatory activity for use in the prevention and/or in the treatment of an inflammatory and/or pre-tumour and/or tumour pathological condition characterised by a constitutive or anomalous activation of the STAT3 transcription factor, said kit comprising one or more aliquots of an extract of *Filipendula vulgaris* as defined in the present description, and one or more aliquots of one or more compounds having anti-tumour activity and/or one or more aliquots of one or more compounds having anti-inflammatory activity.

Alternatively, the kit may comprise one or more aliquots of the composition containing, as active ingredient, an extract of *Filipendula vulgaris* as defined in the present description and one or more aliquots of one or more compounds having anti-tumour activity and/or one or more aliquots of one or more compounds having anti-inflammatory activity.

As described above, such compounds can be chemotherapeutic agents selected for example in the group comprising cisplatinum, doxorubicin, pemetrexed, methotrexate, vinorelbine, gemcitabine and taxol.

The pathologies that can be treated or prevented using the kit or using the composition of the present invention are those already indicated in the description above, pathological states characterised by a constitutive or anomalous activation of the STAT3 transcription factor that can be caused for example by viral infections (as noted in the literature), including infections by *H pylori,* infections by the Hepatitis B virus, infections by HPV (human papilloma virus), infections by the Epstein-Barr virus (as reported in Yu et al 2009), or tumour pathological states that can be represented by any tumour characterised by a constitutive or anomalous activation of STAT3 reported in the prior art.

A non-limiting example of such tumours comprises:
prostate cancer, multiple myeloma, leukaemia, lymphoma, melanoma, carcinoma of the ovaries, carcinoma of the kidney cells, pancreatic adenocarcinoma, lung cancer, brain cancer, erythroleukaemia, squamous-cell carcinoma of the head and neck, colon cancer, mesothelioma.

More specifically, said brain tumour could be, for example, a glioma, a brain meningioma, a medulloblastoma, said lymphoma could be Sezary syndrome, EBV-associated Burkitt lymphoma, Saimiri HSV-dependent lymphoma, cutaneous T-cell related lymphoma; said leukaemia may be HTLV-I-dependent leukaemia, chronic lymphocytic leukaemia (CLL), acute myelogenous leukaemia (AML), megakaryocytic leukaemia, large granular lymphocytic leukaemia (LGL).

The pre-tumour pathological states in which a constitutive or anomalous activation of STAT3 is present can be either pathological states following the ablation of a tumour, and thus pre-tumour in the sense that the tumour could reform, or pathological states in which there is a transfer from inflammation to the acquisition of malignant characteristics on the part of the cell, as reported in the literature.

Lastly, the present description also concerns a therapeutic method for the prevention and/or the treatment of an inflammatory and/or pre-tumour and/or tumour pathological condition characterised by a constitutive or anomalous activation of the STAT3 transcription factor comprising the step of administering to an individual in need of it a therapeutically active quantity of extract of *Filipendula vulgaris* or of a pharmaceutical composition comprising extract of *Filipendula vulgaris* optionally in association with one or more anti-tumour and/or anti-inflammatory compounds.

The method forming the basis of the present invention can be carried out by administering to a subject who presents an inflammatory and/or pre-tumour and/or tumour pathological condition characterised by a constitutive or anomalous activation of the STAT3 transcription factor, therapeutically effective doses of the extract as defined here, optionally in association with one or more anti-tumour or anti-inflammatory drugs; or by administering therapeutically effective doses of the composition as defined here, optionally further comprising one or more anti-tumour and/or anti-inflammatory drugs, or by administering the extract and one or more anti-tumour and/or anti-inflammatory drugs using the kit as defined here.

The administration as described above can be performed concomitantly or sequentially in accordance with the administration regime selected by the doctor.

Numerous experimental data have been reported that demonstrate the efficacy of the extract according to the present invention.

### USED CELL LINES

- L428 Human lymphoma cell line. Available from DSMZ ACC197
- KARPAS Human lymphoma cell line with constitutively activated STAT3. Available from Cell Bank Australia #6072604
   Human T-cell lymphoma cell line, established from peripheral blood of a human of 25 years of age with non-Hodgkin T-cell lymphoma cells in 1986, now classified as lymphoblastoid lymphoma cell line. Karpas 299 expresses Stat3 phosphorylated in tyrosine 705 and serine 727.
- MSTO211H Human lung biphase mesothelioma cell line with constitutively activated STAT3. Available from ATCC #CLR-2081

Human mesothelioma cell line, established from the pleural spill of a human of 62 years of age with mesothelioma (biphase malignant) who had not had any prior therapy. Cell line MSTO211H expresses high levels of pStat3).(Tsao et al. Inhibition of c-Src expression and activation in malignant pleural mesothelioma tissues leads to apoptosis, cell cycle arrest, and decreased migration and invasion. MolCancerTher 2007;6:1962-1972.)
- DU-145 Human carcinoma cell line available from ATCC #HTB-81
   The cell line DU145 is a human prostate cancer cell line of moderate metastatic potential compared with PC3 cells, which have high metastatic potential. The DU145 cells are not hormone-sensitive and do not express PSA (prostate-specific antigen). The cell line DU145 expresses pStat3 in a constitutive manner.
- HCT116 Human colon cancer cell. Available from ATCC #CCL-247.
- MDA-MB-231 Human mammary adenocarcinoma cell line. Available from ATCC #HTB-26.
- NCI-h28 Human stage-4 mesothelioma cell line. Available from ATCC#CRL-5820
- MPP-89 Human mesothelioma cell line. Available from CABRI, access number ICLC HTL00012

### EXAMPLES

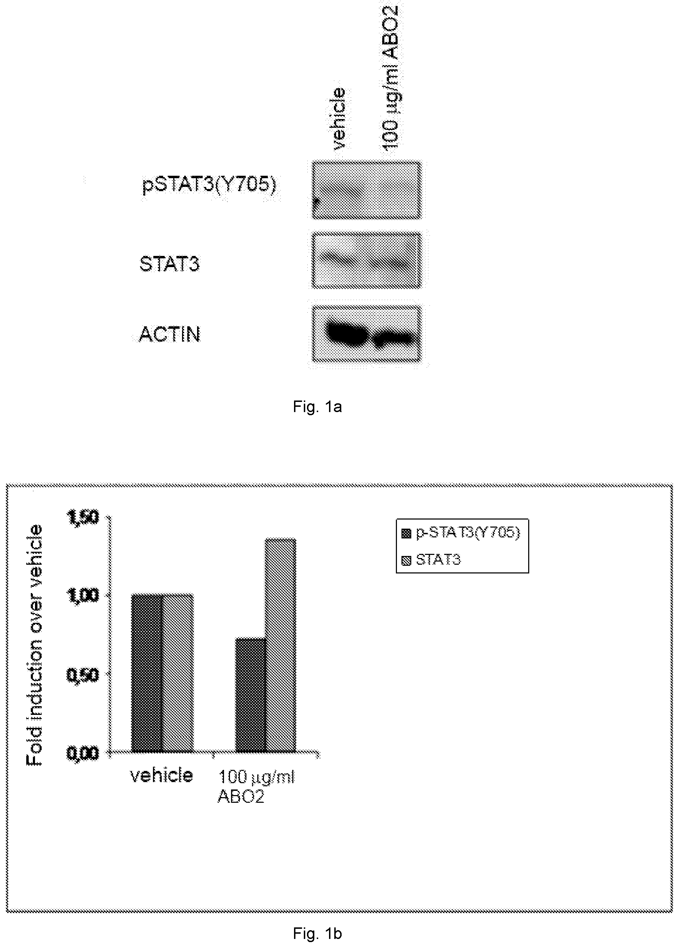

### 1. Analysis of the phosphorylation of STAT3 by means of Western Blot. Results reported in Figures 1-3.

### 1.1. Cell lysis and Western blotting.

The cells were lysed in ice for 30 min in lysis buffer NP40 (50 mM Tris-HCI pH 7.4, 150 mM NaCl, 1% NP-40, 1 mM EGTA, 1 mM EDTA) complemented with inhibitors of protease and phosphatase (5 mM PMSF, 3 mM NaF, 1 mM DTT, 1 mM NaVO4). Equal amounts of total extracts of protein (30 µg) were broken down by means of denaturing electrophoresis (SDS-PAGE) in 8% polyacrylamide gel and transferred for 2 hours on nitrocellulose membrane. The membranes were blocked with a 5% solution of milk dissolved in TBS-Tween_20 0.05% for 1 hour and incubated with the specific primary antibodies. The following primary antibodies were used: anti-beta actin (A-2228, SIGMA), anti-pSTAT3 (Tyr-705) (sc8059, Santa Cruz) and anti-STAT3 (sc7179, Santa Cruz). The secondary antibodies were peroxidase-conjugated (Santa Cruz), and ECL reagents (Amersham, GE Healthcare, Piscataway, NJ, USA) were used for the chemiluminescence.

### 1.2. Treatment of the cell lines of MPMs and of normal commercial mesothelial cells (HMC) with extract of Filipendula vulgaris.

The cell lines of MPMs (MSTO-211H, NCI-H28, NCI-H2052, MPP89) were acquired from ATCC (Rockville, MD) whilst the HMCs (Human Mesothelial Cells) were acquired from Tebu-Bio (France). All the lines were grown in monolayers at 37°C and at 5% of CO2 in specific culture media. The *Filipendula vulgaris* extract was dissolved conveniently in a solution of water for injectable solutions and ethanol in a ratio of 1:1 at an initial concentration of 30mg/ml. To test the anti-tumour property, the product was then added directly in the medium of the various cell lines using various concentrations and various times, as shown in the drawings.

### 1.3. Results

The results, shown in Figures 1 to 3, show how the assayed extract inhibits the phosphorylation of STAT3 compared with the controls not treated with the extract.

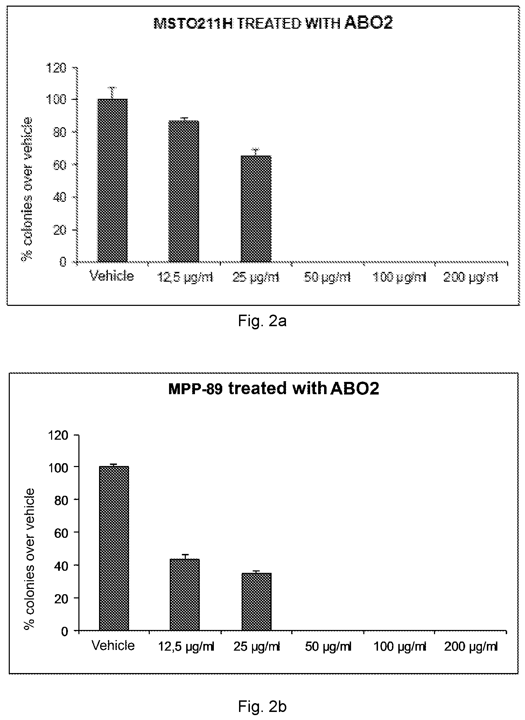

Figures 1 and 2 show the data obtained on MSTO211H cells treated with extract of *Filipendula vulgaris* in accordance with the description.

Figure 1 shows the data with the control treated with just the carrier and extract of *Filipendula vulgaris,* 100 µg/ml of culture medium for 24 hours (actin control).

### 2. Assay of clonogenicity on cells of Malignant Pleural Mesothelioma (MPM)

MPM cells (MSTO211H and MPP-89) were seeded at 200 cells per well and were treated with various growing concentrations (control just with carrier; 12.5 µg/ml; 25 µg/ml; 50 µg/ml; 100 µg/ml, 200 µg/ml) of extract of *Filipendula vulgaris* in accordance with the present description. Each point was plated in duplicate in the 6-well multiwall. The colonies formed were stained with violet crystal 15-21 days later. The assay of colony formation, also known as a clonogenic assay, is a technique used to assess the efficacy of anti-tumour compounds in terms of the ability of the tumour cells to form colonies from a single cell. A colony is considered to be a group of 50 or more cells (clones) originating from a single cell.

The results of the experiment, shown in Figures 2a and 2b, show the dose-dependent ability of the extract of the invention to inhibit, in a dose-dependent manner, the formation of colonies in all the MPM cell lines tested.

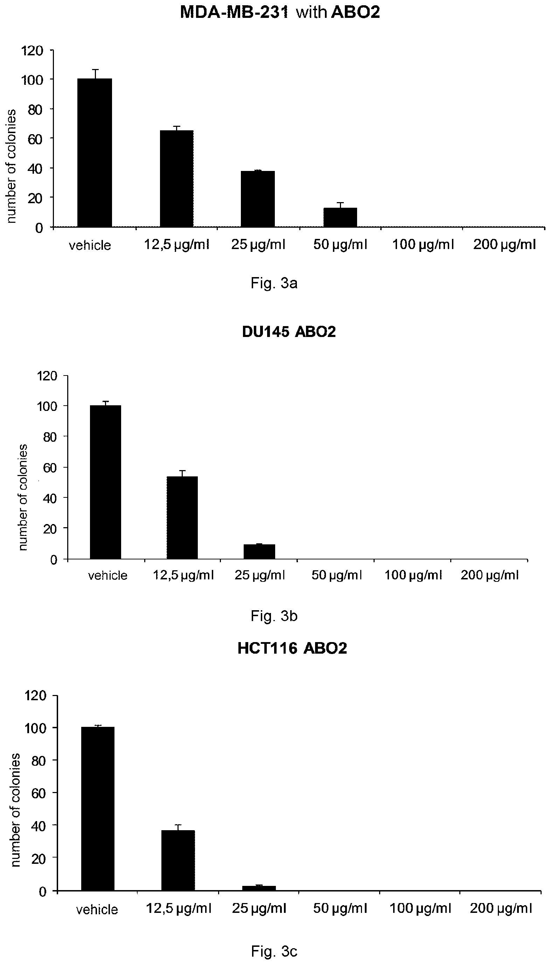

The same assay was also performed on MDA-MB-231 breast cancer cells, DU145 prostate cancer cells and HCT116 colon cancer cells. In this case too, the data shown in Figures 3a, b, and c show the efficacy of inhibiting, in a dose-dependent manner, the formation of colonies from the extract of the invention.

### 4. Assay of cell vitality ATPlite™

The vitality of various cell lines following exposure to the extract of the invention at various concentrations was assessed using the ATPlite™ assay (Perkin Elmer) in accordance with the producer's instructions. Where indicated, the term "carrier" refers to a solution of water for injectable solutions and ethanol at a concentration of 1:1 used in the same volumes used for the treatments.

ATPLite™ is a system for monitoring the levels of adenosine triphosphate (ATP) based on the activity of firefly (Photinus pyralis) luciferase. This luminescence assay is an alternative to colorimetric, fluorometric and radioisotopic tests for the quantitative evaluation of the proliferation of cultured mammalian cells subjected to treatment with possible substances contained in the culture medium. The monitoring of ATP is used in fact to evaluate the cytostatic and anti-proliferative effects of a vast range of drugs, modifiers of the biological response, and biological compounds. The ATPLite™ test system is based on the production of light caused by the reaction with addition of ATP luciferases and D-luciferin. The light emitted is proportional to the concentration of ATP within certain limits. The quantity of ATP in cells correlates with the cell vitality.

The cell vitality of various types of MPM cell lines (MSTO211H, MPP89, NCI-H28) and of HMC cells (untransformed mesothelial cells provided by willing donors) were assayed following treatment with various concentrations of extract according to the invention (control just with carrier; 12.5 µg/ml; 25 µg/ml; 50 µg/ml; 100 µg/ml, 200 µg/ml).

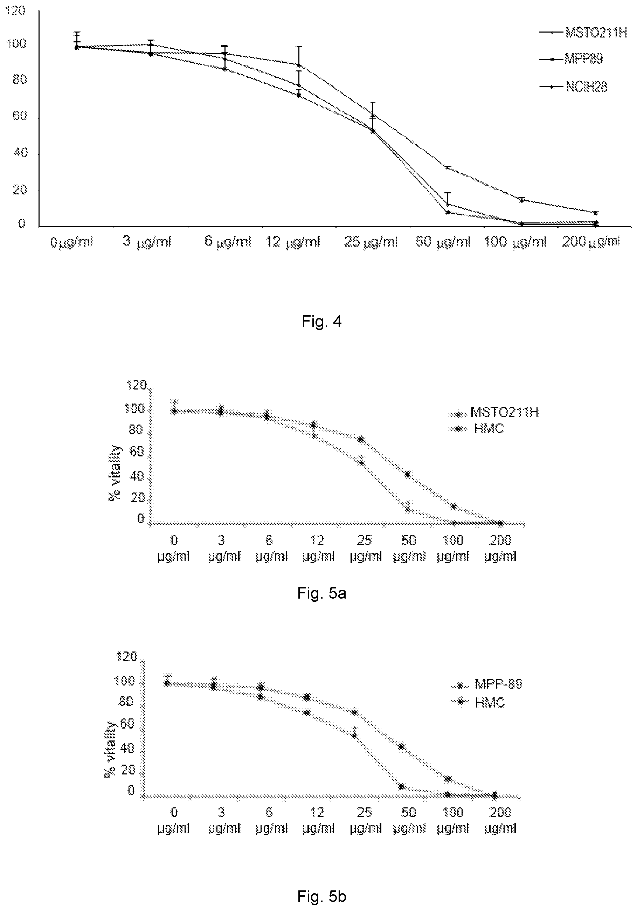

The graph in Figure 4, which shows the results of the assay, shows that the extract is able to significantly reduce cell vitality in a dose-dependent manner.

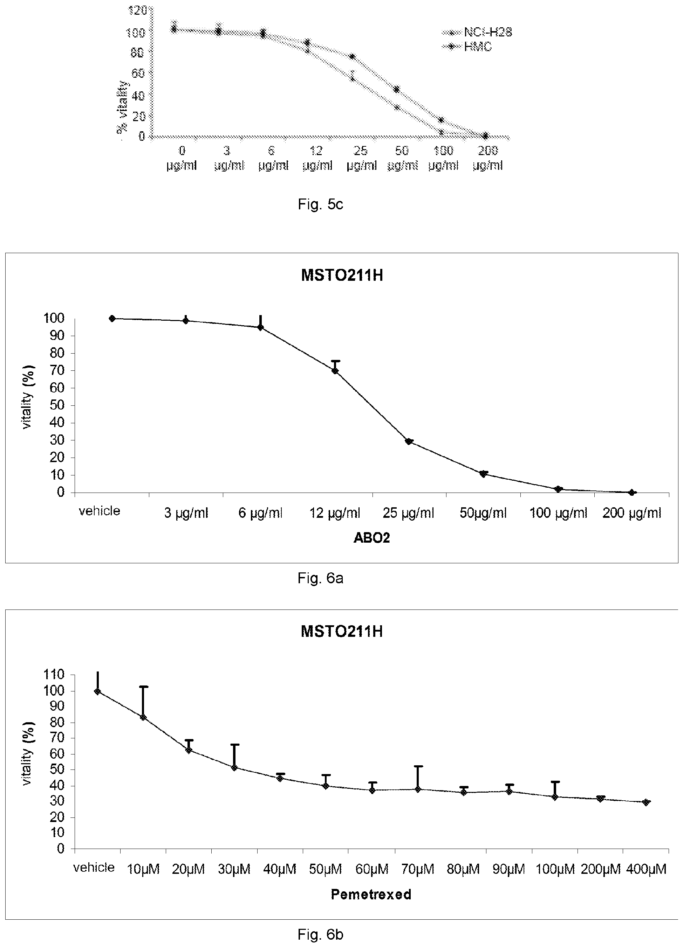

The effects on cell vitality were also tested on untransformed mesothelial cells (HMCs), towards which the extract forming the basis of the invention demonstrated lower cytotoxicity compared with the tumour lines (figures 5A - 5C).

### 7. Assays of cell vitality in co-treatment with chemotherapeutic agents

Cell lines MSTO211H and NCI-H2052 were used to evaluate the effects of the association of extract of *Filipendula vulgaris*₋+ anti-tumour drug.

The assay shown in Figure 10 was performed using ATPlite™ assay (Perkin Elmer) in accordance with the producer's instructions.

A solution of water for injectable solutions and ethanol at a concentration of 1:1 was used in the same volumes used for the treatments.

Reagents:
pemetrexed (Alimta, Lilly) diluted in accordance with the producer's instructions.

### 7.1 Association of extract of Filipendula vulgaris and pemetrexed with ATPlite™ assay

Figure 9 shows the vitality curve for MSTO211H after 72h of treatment with pemetrexed and pemetrexed in association with extract of *Filipendula vulgaris.* Graph A shows the treatment with the extract at non-cytotoxic dose (6 µg/ml) and pemetrexed for the MSTO211H cells, whereas graph B shows the treatment with the extract at non-cytotoxic dose (6 µg/ml) and with pemetrexed (various concentrations) for NCI-H2052 cells, and graph C shows the treatment with the extract at non-cytotoxic dose (6 µg/ml) and with pemetrexed (various concentrations) for untransformed HMC cells. The concentrations of the assayed compound are plotted on the abscissa, whereas the cell vitality expressed in percentage is plotted on the ordinate.

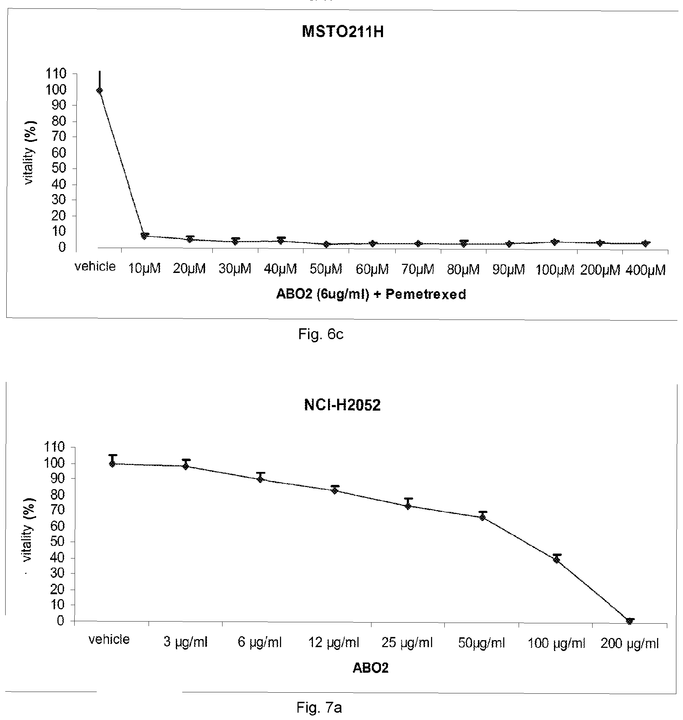

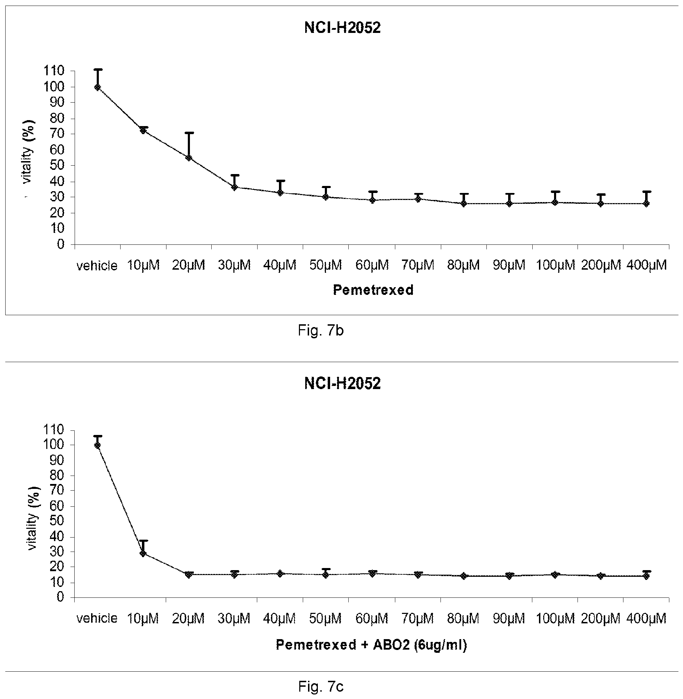

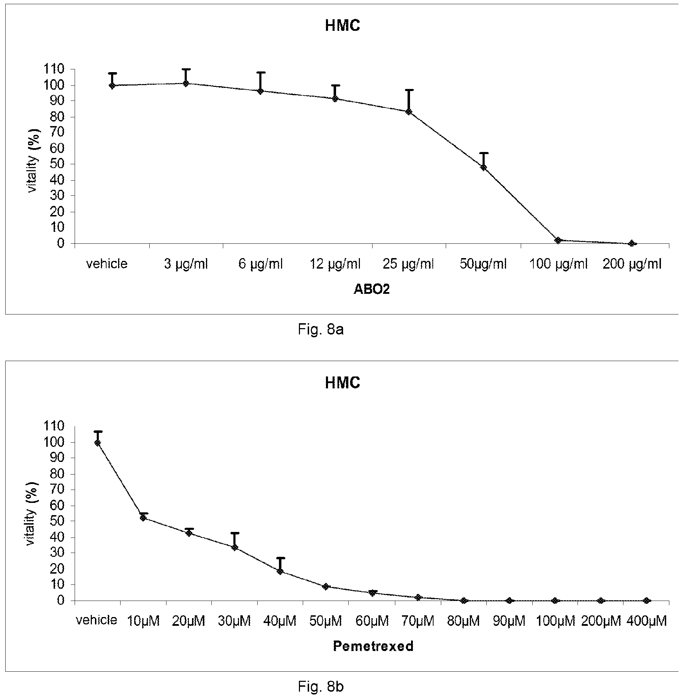

Figures 7 and 8 show how the treatment with extract sensitises the tumour lines to the treatment with pemetrexed. In the curve with double treatment, it is clear how just a concentration of pemetrexed of 10 µM is sufficient to lower the cell vitality of the tested lines. It is interesting to note that, in the non-tumour line (see Figure 8) the extract has a protective effect towards pemetrexed.

### 8. Woundhealing assay

The woundhealing assay (Figure 9) is simple, inexpensive, and one of the first methods developed for studying directional cell migration *in vitro.* This method mimics cell migration during would healing *in vivo.* The basic steps involve creating a "wound" in a cell monolayer, then monitoring a specific zone of the "wound" by capturing images at the beginning and at regular intervals during the cell migration necessary to close the "wound". The MSTO211H cells cultivated with a confluency of 95 % were seeded in 6-well textile plates and the "wound" (or cut) was made with a puncture by 10-microlitre sterile pipette to remove the cells. Digital micrographs were produced after the wounds at the indicated times. The final bar chart shows the efficacy of closure of the cut (quantification number of the cells in %) treated with carrier or ABO 1 at the indicated times.

### 9. Assay to assess the induction of apoptosis

### See Figure 10

### 9.1 Western blotting

The same technique as described in point 1.1 was used, and the following primary antibodies were used: anti-beta actin (A-2228, SIGMA), anti-caspase-3 (31A1067, Alexis), anti-caspase-7 (#9492, Cell Signalling) and anti-PARP (#9542S, Cell Signalling).

### 9.2 FACS analysis, PI staining and PI/Annexin V staining analyses

For the purpose of determining the effect of the extract of the invention on the cell cycle, a FACS analysis was performed.

For staining with propidium iodide (PI), the cells were seeded in 6-well plates at a density of 10⁴ cells/ml. After 24 h, the tumour cells were treated with indicated concentrations of the extract of the invention for various time intervals. The cells were collected in suspension and the adhered cells were washed in PBS, fixed with frozen ethanol (70% v/v) and stored at -20 °C. For the analyses, the cells were washed in PBS 1X and suspended in a solution of PBS 1Z, PI (25 mg / ml) and RNase A (200 mg/ml).

For the PI/annexin V double staining, the treated cells were collected and resuspended in binding buffer (HEPES pH 7.4, CaCl2 2.5 mM, NaCl 140 mM). Aliquots of cells were incubated for 15 min with annexin V FITC and PI (5 mg/mL) (Invitrogen).

During all the FACS analyses, 10⁵ events were analysed for each sample. The flow cytometry analyses were performed on a GuavaEasyCyte 8HT (Millipore) flow cytometer.

As can be seen in Figure 11, the extract of the invention induces apoptosis in MSTO211H cells, as determined by the annexin V staining, in a time-dependent and dose-dependent manner.

### 12. Transplantation of tumour cells treated or untreated with the extract of the invention

### Description of the first engraftment experiment.

The MSTO211H cells were treated with *Filipendula vulgaris* at the concentration of 50 µg/ml for 24 hours. A suspension of 2 x 10⁶ of cells in PBS/Matrigel (BD Biosciences) was collected and inoculated in the right hip of nude female mice 4 weeks old. The volume of the tumours was monitored twice a week up to the 21^{st} day. The mice were sacrificed and the masses removed.

### 13. Transplantation of tumour cells in mice and treatment with Filipendula vulgaris and pemetrexed

### Description of the second engraftment experiment.

The cells were expanded prior to the implantation and were evaluated in terms of their vitality and contamination, that is to say were counted and resuspended in PBS at a concentration of 20 x10⁶/ml. Matrigel was added to the suspension to obtain a final concentration of 10x10⁶ cells/ml of PBS Matrigel 1/1. The MSTO cells were inoculated under the skin in 48 mice.

When the tumour reached an average volume of 60 mm³, the mice were divided into 8 groups formed by 6 animals per group, receiving different treatments.

Two groups received *Filipendula vulgaris* in drinking water for 7 days of the week during a period of three weeks; the other groups received pemetrexed intraperitoneally for 5 days of the week during a period of 3 weeks.

The groups have been outlined in this way in Table 5 below:

| | no. animals | cell line | no. cells | pathway | volume | treatm. A | start of treatm. | administrat. method | treatm. regime | treatm. B | start of treatm. | administrat. method | treatm. regime |
|---|---|---|---|---|---|---|---|---|---|---|---|---|---|
| Group 1 | 6 | MSTO | 2X10⁶ | SC | 0.2 ml (matrigel) | | | | | Filipendula extract 20µg/ml | after tumour appearance | OS | drinkin water |
| Group 2 | 6 | MSTO | 2X10⁶ | SC | 0.2 ml (matrigel) | | | | | Filipendula extract 50µg/ml | after tumour appearance | OS | drinkin water |
| Group 3 | 6 | MSTO | 2X10⁶ | SC | 0.2ml (matrigel) | | | | | Filipendula extract 750µg/ml | after tumour appearance | OS | drinking water |
| Group 4 | 6 | MSTO | 2X10⁶ | SC | 0.2 ml (matrigel) | | | | | | | | |
| Group 5 | 6 | MSTO | 2X10⁶ | SC | 0.2 ml (matrigel) | Pemetrexed (100mg/kg) | aftertumour appearance | IP | 5 days in succession | Filipendula extract 20µg/ml | after tumour appearance | OS | drinking water |
| Group 6 | 6 | MSTO | 2X10⁶ | SC | 0.2 ml (matrigel) | Pemetrexed (100mg/kg) | aftertumour appearance | IP | 5 days in succession | Filipendula extract 50µg/ml | after tumour appearance | OS | drinking water |
| Group 7 | 6 | MSTO | 2X10⁶ | SC | 0.2 ml (matrigel) | Pemetrexed (100mg/kg) | aftertumour appearance | IP | 5 days in succession | Filipendula extract 75µg/ml | after tumour appearance | OS | drinking water |
| Group 8 | 6 | MSTO | 2X10⁶ | SC | 0.2 ml (matrigel) | Pemetrexed (100mg/kg) | aftertumour appearance | IP | 5 days in succession | | | | |

| | | | | | | | | | | | | | |
|---|---|---|---|---|---|---|---|---|---|---|---|---|---|
| SC = subcutaneous treatm. = treatment administrat. = administration IP = intraperitoneal OS = oral | | | | | | | | | | | | | |

With appearance of progression of the tumour (that is to say when the tumour reached 60 mm³), treatment was started with ABO2 and Pemetrexed administered as follows: pemetrexed at a dose of 100 mg/Kg in 88 ml/mouse for 5 consecutive days intraperitoneally) *Filipendula vulgaris* extract in drinking water at concentrations of 25, 50 and 75 micrograms/ml and measured on alternate days for a period of 3 weeks.

The mice were monitored daily to evaluate any signs; body weight was monitored twice weekly.

At the end of the experiment (42 days after inoculation), the tumour masses were collected and fixed in 10 % formalin (transferred after 24 hours to 70 % ethanol).

The tumour diameters were measured twice weekly using a Mitutoyo caliper.

### 14. Preparation of Filipendula vulgaris extract

*Filipendula vulgaris* leafy inflorescences, previously cut to a correct grain size, were subjected to hydroalcoholic extraction with 50% ethanol.

The extraction was performed at 50 °C for 8 hours. At the end of the extraction, spent leafy inflorescences were removed from the hydroalcoholic solution obtained by filtration. The solution was concentrated by thin-film evaporation until complete ethanol removal. The concentrated aqueous solution thus obtained was dried in a lyophiliser until obtaining a solid cake.

Lastly, the lyophilised cake was powdered using a hammer mill and mixed to obtain a homogeneous powdered lyophilised extract.

### 15. Cell lines and culture conditions.

Cell lines MSTO-211H, NCI-H28, NCI-H2052 MPP89 were obtained from ATCC (Rockville, MD). HMC (Human Mesothelial Cells) lines were obtained from Tebu-Bio (France). All cell lines were kept in a humidified incubator at 37°C in 5% CO₂. The cells were cultured as monolayer in DMEM/F12 + GLUTAMAX (INVITROGEN) complemented with 10% not heat-inactivated FBS (GIBCO), 5 mgr/mL insulin (SIGMA), 100 IU/ml penicillin G sodium and 100 mg/ml streptomycin sulphate.

### 16. Cell reagents

Pemetrexed (ALIMTA, Lilly) and Cisplatinum (Pfizer) were dissolved in accordance with the producer's instructions.

### 17 Comet assay

After the treatment, the cells were trypsinized and immersed in 1% low-melting agarose (Sigma) in PBS and spread onto microscope slides coated beforehand with 1% agarose (Bio-Rad). The cells were lysed in the lysis solution (2.5 M NaCl, 100 mM EDTA, 10 mM Tris base, 8 g/l NaOH, 1% Triton X-100, 10% DMSO) for 1 hour at room temperature and then run into the running solution (300 mM NaOH, 1 mM EDTA, pH 13.0) for 30 minutes, at 25 V and 250 mA. DNA was balanced with 0.4 M Tris, pH 8.0, and the slides dried with methanol. Propidium iodide-DNA (Sigma) and the figures images were taken by using an Axiovert 200M microscope with 40X magnification and an Axiovision (Zeiss) image acquisition software. At least 300 cells per each slide were recorded.

### 18. FACS analysis and PI staining and PI/Annexin V staining analyses

For the purpose of determining the effect of the extract of the invention on the cell cycle, a FACS analysis was performed.

For staining with propidium iodide (PI), the cells were seeded in 6-well plates at a density of 10⁴ cells/ml. After 24 h of adhesion, the tumour cells were treated with indicated concentrations of the extract of the invention for various time intervals. The cells were collected in suspension and the adhered cells were washed in PBS, fixed with frozen ethanol (70% v/v) and stored at -20 °C. For the analyses, the cells were washed in PBS and suspended in PBS, PI (25 mg / ml) and RNase A (200 mg/ml).

For the PI/annexin V double staining, the treated cells were collected and resuspended in binding buffer (HEPES pH 7.4, CaCl2 2.5 mM, NaCl 140 mM). Aliquots of cells were incubated per 15 min with annexin V FITC and PI (5 mg/mL) (Invitrogen).

During all the FACS analyses, 10⁵ events were analysed for each sample. The flow cytometry analyses were performed on a GuavaEasyCyte 8HT (Millipore) flow cytometer.

### 19. Statistical analysis

Student's t-test was used to evaluate the significance of data *in vitro,* whilst the significance of survival data on mice was evaluated using Kaplan-Meier analysis and Log Rank tests. p ≤ 0.05 values were considered statistically significant.

### Xenograft Transplantation

Suspensions of 1 x 10⁶ MSTO211H cells in PBS 1x/ Matrigel (BD Biosciences) were subcutaneously injected in male 5-week CD1 mice (Charles River, Milan). Mice body weight and clinical signs were determined every 3 days. Tumour volumes were calculated using the formula: V = 1/2 x length x width 2 (length and width were measured with a manual caliper). When the tumours reached a volume of 50mm³, the mice were treated intraperitoneally with pemetrexed (100 mg/kg/3 days) or orally with *Filipendula vulgaris* extract (25/50/100 µg/ml/7 d). All tumourigenity assays were performed following Internal Ethics Committee guidelines.

### Western blotting

The cells were lysed in ice for 30 min in lysis buffer NP40 (50 mM Tris-HCI pH 7.4, 150 mM NaCl, 1% NP-40, 1 mM EGTA, 1 mM EDTA) complemented with inhibitors of protease and phosphatase (5 mM PMSF, 3 mM NaF, 1 mM DTT, 1 mM NaVO4).

Equal amounts of total extracts of protein (30 µg) were broken down by means of denaturing electrophoresis (SDS-PAGE) in 8% polyacrylamide gel and transferred for 2 hours on pure nitrocellulose membrane. The membranes were blocked with 5% milk -TBS-0.05% Tween 20 for 1 hour and incubated overnight with the specific primary antibodies. The following primary antibodies were used: anti-beta actin (A-2228, SIGMA), anti-Caspase-3 (31A1067, Alexis); anti-Caspase-7 (#9492, Cell Signaling); anti-PARP (#9542S, Cell Signaling). The secondary antibodies used for chemiluminescence detection were radish peroxidase-conjugated (Santa Cruz), and ECL reagents (Amersham, GE Healthcare, Piscataway, NJ, USA) were used for the chemiluminescence.

### BIBLIOGRAPHY

- Aggarwal B. B. et al. Ann. N.Y. Acad. Sci. 1091; 151-69: 2006
- Johnston PA and Grandis RG, Mollnterv; 11 (1); 18-26:2011
- Niu G. et al. Mol Cancer Res, 6 (7); 1099-105: 2008
- Turkson J. Jove R. "STAT proteins: novel molecular targets for cancer drug discovery" Oncogene. 2000 Dec 27;19(56):6613-26
- Yu.H. et al "STATs in cancer inflammation and immunity: a leading role for STAT3" Nature Reviews Cancer 9, 798-809 (November 2009)

## Claims

1. A *Filipendula vulgaris* extract for use in the prevention and/or in the treatment of an inflammatory and/or pre-tumour and/or tumour pathological condition **characterised by** a constitutive or anomalous activation of the STAT3 transcription factor.

2. The *Filipendula vulgaris* extract according to claim 1 wherein said extract is a dry or lyophilised or fluid extract of leafy inflorescences of *Filipendula vulgaris.*

3. The *Filipendula vulgaris* extract according to any one of claims 1 or 2 for use in the prevention and/or treatment of an inflammatory and/or pre-tumour and/or tumour pathological condition **characterised by** a constitutive or anomalous activation of the STAT3 transcription factor in association with one or more anti-tumour and/or anti-inflammatory compounds. wherein said association is carried out by concomitant or sequential administration of said extract with said one or more anti-tumour and/or anti-inflammatory compounds.

4. The *Filipendula vulgaris* extract according to claim 3 wherein said one or more anti-tumour compounds are selected in the group comprising: cisplatinum, doxorubicin, pemetrexed, methotrexate, vinorelbine, gemcitabine and taxol.

5. The *Filipendula vulgaris* extract according to any one of claims from 1 to 4 wherein said pathological condition is a tumour pathological condition selected in the group comprising: prostate cancer, multiple myeloma, leukaemia, lymphoma, melanoma, ovary carcinoma, kidney cell carcinoma, pancreatic adenocarcinoma, lung cancer, brain cancer, erythroleukaemia, squamous-cell carcinomas of the head and neck, colon cancer, mesothelioma or wherein condition is an inflammation caused by viral infections such as infection by *H pylori,* infection with hepatitis B virus, infections by HPV (human papilloma virus), Epstein-Barr virus infections.

6. The *Filipendula vulgaris* extract according to claim 5 wherein said brain tumour is glioma, brain menangioma, medulloblastoma, wherein said lymphoma is Sezary syndrome, EBV associated Burkitt lymphoma, Saimiri HSV-dependent lymphoma, cutaneous T-cell related lymphoma; wherein said leukaemia is HTLV-I-dependent leukaemia, chronic lymphocytic leukaemia (CLL), acute myelogenous leukaemia (AML), megakaryocytic leukaemia, large granular lymphocytic leukaemia (LGL).

7. The *Filipendula vulgaris* extract according to any one of claims from 1 to 6, wherein said pathological condition is a tumour resistant to treatment with chemotherapeutic agents that do not inhibit STAT3.

8. A composition comprising as active principle a *Filipendula vulgaris* extract as defined in claims 1 or 2, and a carrier and/or diluent and/or excipient for use in the prevention and/or treatment of an inflammatory and/or pre-tumour and/or tumour pathological condition **characterised by** a constitutive or anomalous activation of the STAT3 transcription factor optionally further comprising, as active principle, one or more anti-tumour and/or anti-inflammatory compounds.

9. The composition for use according to claim 8 wherein said one or more anti-tumour compounds are selected in the group comprising: cisplatinum, doxorubicin, pemetrexed, methotrexate, vinorelbine, gemcitabine and taxol.

10. The composition for use according to any one of claims 8 or 9 wherein said pathological condition is a tumour pathological condition selected in the group comprising: prostate cancer, multiple myeloma, leukaemia, lymphoma, melanoma, ovary carcinoma, kidney cell carcinoma, pancreatic adenocarcinoma, lung cancer, brain cancer, erythroleukaemia, squamous cell carcinomas of the head and neck, colon cancer, mesothelioma or wherein said condition is an inflammation caused by viral infections such as infection by *H pylori,* infection with hepatitis B virus, infections by HPV (human papilloma virus), Epstein-Barr virus infections.

11. The composition for use according to claim 10 wherein said brain tumour is glioma, brain menangioma, medulloblastoma, wherein said lymphoma is Sezary syndrome, EBV associated Burkitt lymphoma, Saimiri HSV-dependent lymphoma, cutaneous T-cell related lymphoma; wherein said leukaemia is HTLV-I-dependent leukaemia, chronic lymphocytic leukaemia (CLL), acute myelogenous leukaemia (AML), megakaryocytic leukaemia, large granular lymphocytic leukaemia (LGL).

12. The composition for use according to any one of claims from 8 to 11, wherein said pathological condition is a tumour resistant to treatment with chemotherapeutic agents that do not inhibit STAT3.

13. A kit for the concomitant or sequential administration of *Filipendula vulgaris* extract and one or more compounds having anti-tumour activity and/or one or more compounds having anti-inflammatory activity for use in the prevention and/or treatment of an inflammatory and/or pre-tumour and/or tumour pathological condition **characterised by** a constitutive or anomalous activation of the STAT3 transcription factor, comprising one or more aliquots of a *Filipendula vulgaris* extract as defined in claims 1 or 2, and one or more aliquots of one or more anti-tumour compounds and/or one or more aliquots of one or more anti-inflammatory compounds.

14. The kit according to claim 13, wherein said one or more anti-tumour compounds are selected in the group comprising cisplatinum, doxorubicin, pemetrexed, methotrexate, vinorelbine, gemcitabine and taxol.

15. The kit according to any one of claims 17 or 18, wherein said pathological condition is a tumour pathological condition selected in the group comprising: prostate cancer, multiple myeloma, leukaemia, lymphoma, melanoma, ovary carcinoma, kidney cell carcinoma, pancreatic adenocarcinoma, lung cancer, brain cancer, erythroleukaemia, squamous cell carcinomas of the head and neck, colon cancer, mesothelioma.

## Patentansprüche

1. *Filipendula* vulgaris-Extrakt zur Verwendung bei der Vorbeugung und/oder bei der Behandlung einer Entzündungs- und/oder Turmorvorstufen- und/oder Tumor-bedingten pathologischen Bedingung, **gekennzeichnet durch** eine konstitutive oder anormale Aktivierung des STAT3-Transcriptionsfaktors.

2. *Filipendula* vulgaris-Extrakt gemäß Anspruch 1, wobei der Extrakt ein trockener oder lyophilisierter oder flüssiger Extrakt von blattreichen Blütenständen von *Filipendula vulgaris* ist.

3. *Filipendula vulgaris*-Extrakt gemäß einem der Ansprüche 1 oder 2 zur Verwendung bei der Vorbeugung und/oder Behandlung einer Entzündungs- und/oder Tumorvorstufen- und/oder Tumor-bedingten pathologischen Bedingungen, **gekennzeichnet durch** eine konstitutive oder anormale Aktivierung des STAT3-Transcriptionsfaktors im Zusammenhang mit einer oder mehreren Anti-Tumor- und/oder Anti-Entzündungsverbindungen, wobei der Zusammenhang durch eine gleichzeitige oder sequentielle Verabreichung des Extrakts mit der einen oder den mehreren Anti-Tumor- und/oder Anti-Entzündungsverbindungen hergestellt wird.

4. *Filipendula vulgaris*-Extrakt gemäß Anspruch 3, wobei die eine oder mehreren Anti-Tumor-Verbindungen aus der folgenden Gruppe ausgewählt werden: Cisplatinum, Doxorubicin, Pemetrexed, Methotrexat, Vinorelbin, Gemcitabin und Taxol.

5. *Filipendula vulgaris*-Extrakt gemäß einem der Ansprüche 1 bis 4, wobei die pathologische Bedingung eine pathologische Tumor-bedingte Bedingung ist, die aus der folgenden Gruppe ausgewählt wird: Prostatakrebs, multiples Myelom, Leukämie, Lymphom, Melanom, Eierstockkarzinom, Nierenzellkarzinom, Pankreaskarzinom, Lungenkrebs, Gehirnkrebs, Erythroleukämie, Plattenepithelkarzinom des Kopfes und Halses, Darmkrebs, Mesotheliom, oder wobei die Bedingung eine Entzündung ist, die durch virale Infektionen verursacht wird, wie zum Beispiel eine Infektion durch *H. pylori,* eine Infektion mit dem Hepatitis-B-Virus, Infektionen durch HPV (Humane Papillomviren), Infektionen mit dem Epstein-Barr-Virus.

6. *Filipendula vulgaris*-Extrakt gemäß Anspruch 5, wobei der Gehirntumor umfasst: Gliom, Gehirn-Meningiom, Medullablastom, wobei das Lymphom umfasst: Sezary-Syndrom, EBV-verknüpftes Burkitt-Lymphom, Herpesvirus Saimiri (HSV)-abhängiges Lymphom, kutane T-Zell-Lymphome; wobei die Leukämie umfasst: HTLV-I-abhängige Leukämie, chronische lymphatische Leukämie (CLL), akute myeloische Leukämie (AML), Megakaryozytische Leukämie, Leukämie mit großen granulären Lymphozyten (LGL).

7. *Filipendula vulgaris*-Extrakt gemäß einem der Ansprüche 1 bis 6, wobei die pathologische Bedingung ein Tumor ist, der gegenüber einer Behandlung mit chemotherapeutischen Agenzien, die STAT3 nicht hemmen, resistent ist.

8. Zusammensetzung, die als Wirkstoff umfasst: ein Filipendula vulgaris-Extrakt gemäß den Ansprüchen 1 oder 2 und einen Träger und/oder ein Verdünnungsmittel und/oder einen Hilfsstoff zur Verwendung bei der Vorbeugung und/oder Behandlung einer Entzündungs- und/oder Turmorvorstufen- und/oder Tumor-bedingten pathologischen Bedingung, **gekennzeichnet durch** eine konstitutive oder anormale Aktivierung des STAT3-Transcriptionsfaktors, wobei sie optional weiterhin als Wirkstoff umfasst: eine oder mehrere Anti-Tumor- und/oder Anti-Entzündungsverbindungen.

9. Zusammensetzung zur Verwendung gemäß Anspruch 8, wobei die eine oder mehreren Anti-Tumorverbindungen aus der folgenden Gruppe ausgewählt werden: Cisplatinum, Doxorubicin, Pemetrexed, Methotrexat, Vinorelbin, Gemcitabin und Taxol.

10. Zusammensetzung zur Verwendung gemäß einem der Ansprüche 8 oder 9, wobei die pathologische Bedingung eine pathologische Tumor-bedingte Bedingung ist, die aus der folgenden Gruppe ausgewählt wird: Prostatakrebs, multiplex Myelom, Leukämie, Lymphom, Melanom, Eierstockkarzinom, Nierenzellkarzinom, Pankreaskarzinom, Lungenkrebs, Gehirnkrebs, Erythroleukämie, Plattenepithelkarzinom des Kopfes und Halses, Darmkrebs, Mesotheliom, oder wobei die Bedingung eine Entzündung ist, die durch virale Infektionen verursacht wird, wie zum Beispiel eine Infektion durch H. *pylori,* eine Infektion mit dem Hepatitis-B-Virus, Infektionen durch HPV (humane Papillomviren), Infektionen mit dem Epstein-Barr-Virus.

11. Zusammensetzung zur Verwendung gemäß Anspruch 10, wobei der Gehirntumor umfasst: Gliom, Gehirn-Meningiom, Medullablastom, wobei das Lymphom umfasst: Sezary-Syndrom, EBV-verknüpftes Burkitt-Lymphom, Herpesvirus Saimiri (HSV)-abhängiges Lymphom, kutane T-Zell-Lymphome; wobei die Laukämie umfasst: HTLV-I-abhängige Leukämie, chronische lymphatische Leukämie (CLL), akute myeloische Leukämie (AML), Megakaryozytische Leukämie, Leukämie mit großen granulären Lymphozyten (LGL).

12. Zusammensetzung zur Verwendung gemäß einem der Ansprüche 8 bis 11, wobei die pathologische Bedingung ein Tumor ist, der gegenüber einer Behandlung mit chemotherapeutischen Agenzien, die STAT3 nicht hemmen, resistent ist.

13. Kit zur gleichzeitigen oder sequentiellen Verabreichung von *Filipendula vulgaris*-Extrakt und einer oder mehreren Verbindungen, die über eine Anti-Tumor-Aktivität verfügen, und einer oder mehreren Verbindungen, die über eine entzündungshemmende Aktivität verfügen, zur Verwendung bei der Vorbeugung und/oder Behandlung einer Entzündungs- und/oder Tumorvorstufen- und/oder Tumor-bedingten pathologischen Bedingung, **gekennzeichnet durch** eine konstitutive oder anormale Aktivierung des STAT3-Transcriptionsfaktors, das umfasst: eine oder mehrere Aliquote eines *Filipendula vulgaris*-Extrakts gemäß den Ansprüchen 1 oder 2 und eine oder mehrere Aliquote einer oder mehrerer Anti-Tumor-Verbindungen und/oder eine oder mehrere Aliquote einer oder mehrerer Anti-Entzündungsverbindungen.

14. Kit gemäß Anspruch 13, wobei die eine oder mehreren Anti-Tumor-Verbindungen aus der folgenden Gruppe ausgewählt werden: Cisplatinum, Doxorubicin, Pemetrexed, Methotrexat, Vinorelbin, Gemcitabin und Taxol.

15. Kit gemäß einem der Ansprüche 17 oder 18, wobei die pathologische Bedingung eine pathologische Tumor-bedingte Bedingung ist, die aus der folgenden Gruppe ausgewählt wird: Prostatakrebs, multiplex Myelom, Leukämie, Lymphom, Melanom, Eierstockkarzinom, Nierenzellkarzinom, Pankreaskarzinom, Lungenkrebs, Gehirnkrebs, Erythroleukämie, Plattenepithelkarzinom des Kopfes und Halses, Darmkrebs, Mesotheliom.

## Revendications

1. Extrait de *Filipendula vulgaris* pour une utilisation dans la prévention et/ou dans le traitement d'une affection pathologique inflammatoire et/ou pré-tumorale et/ou tumorale **caractérisée par** une activation constitutive ou anormale du facteur de transcription STAT3.

2. Extrait de *Filipendula vulgaris* selon la revendication 1, où ledit extrait est un extrait sec ou lyophilisé ou liquide des inflorescences feuillues de *Filipendula vulgaris.*

3. Extrait de *Filipendula vulgaris* selon l'une quelconque des revendications 1 ou 2 pour une utilisation dans la prévention et/ou le traitement d'une affection pathologique inflammatoire et/ou pré-tumorale et/ou tumorale **caractérisée par** une activation constitutive ou anormale du facteur de transcription STAT3 en association avec un ou plusieurs composés antitumoraux et/ou anti-inflammatoires, où ladite association est réalisée par une administration simultanée ou séquentielle dudit extrait avec lesdits un ou plusieurs composés antitumoraux et/ou anti-inflammatoires.

4. Extrait de *Filipendula vulgaris* selon la revendication 3 où lesdits un ou plusieurs composés antitumoraux sont choisis dans le groupe comprenant : le cisplatine, la doxorubicine, le pémétrexed, le méthotrexate, la vinorelbine, la gemcitabine et le taxol.

5. Extrait de *Filipendula vulgaris* selon l'une quelconque des revendications 1 à 4 où ladite affection pathologique est une affection pathologique tumorale choisie dans le groupe comprenant : le cancer de la prostate, le myélome multiple, la leucémie, le lymphome, le mélanome, le carcinome de l'ovaire, le carcinome des cellules rénales, l'adénocarcinome pancréatique, le cancer du poumon, le cancer du cerveau, l'érythroleucémie, les carcinomes à cellules squameuses de la tête et du cou, le cancer du côlon, le mésothéliome ou bien où l'affection est une inflammation provoquée par des infections virales telles qu'une infection par *H. pylori,* une infection par le virus de l'hépatite B, des infections par les HPV (papillomavirus humains), des infections par le virus d'Epstein-Barr.

6. Extrait de *Filipendula vulgaris* selon la revendication 5 où ladite tumeur du cerveau est un gliome, un méningiome cérébral, un médulloblastome, où ledit lymphome est le syndrome de Sézary, le lymphome de Burkitt associé à l'EBV, le lymphome dépendant du HSV saimiri, le lymphome cutané associé aux lymphocytes T ; où ladite leucémie est la leucémie dépendante du HTLV-I, la leucémie lymphocytaire chronique (LLC), la leucémie myélogène aiguë (LMA), la leucémie mégacaryocytaire, la leucémie à grands lymphocytes granuleux (GLG).

7. Extrait de *Filipendula vulgaris* selon l'une quelconque des revendications 1 à 6, où ladite affection pathologique est une tumeur résistante à un traitement avec des agents chimiothérapeutiques qui n'inhibent pas STAT3.

8. Composition comprenant en tant que principe actif un extrait de *Filipendula vulgaris* tel que défini dans les revendications 1 ou 2, et un support et/ou un diluant et/ou un excipient pour une utilisation dans la prévention et/ou le traitement d'une affection pathologique inflammatoire et/ou pré-tumorale et/ou tumorale **caractérisée par** une activation constitutive ou anormale du facteur de transcription STAT3 comprenant éventuellement en outre, en tant que principe actif, un ou plusieurs composés antitumoraux et/ou anti-inflammatoires.

9. Composition pour une utilisation selon la revendication 8 dans laquelle lesdits un ou plusieurs composés antitumoraux sont choisis dans le groupe comprenant : le cisplatine, la doxorubicine, le pémétrexed, le méthotrexate, la vinorelbine, la gemcitabine et le taxol.

10. Composition pour une utilisation selon l'une quelconque des revendications 8 ou 9 où ladite affection pathologique est une affection pathologique tumorale choisie dans le groupe comprenant : le cancer de la prostate, le myélome multiple, la leucémie, le lymphome, le mélanome, le carcinome de l'ovaire, le carcinome des cellules rénales, l'adénocarcinome pancréatique, le cancer du poumon, le cancer du cerveau, l'érythroleucémie, les carcinomes à cellules squameuses de la tête et du cou, le cancer du côlon, le mésothéliome ou bien où l'affection est une inflammation provoquée par des infections virales telles qu'une infection par *H. pylori,* une infection par le virus de l'hépatite B, des infections par les HPV (papillomavirus humains), des infections par le virus d'Epstein-Barr.

11. Composition pour une utilisation selon la revendication 10 où ladite tumeur du cerveau est un gliome, un méningiome cérébral, un médulloblastome, où ledit lymphome est le syndrome de Sézary, le lymphome de Burkitt associé à l'EBV, le lymphome dépendant du HSV saimiri, le lymphome cutané associé aux lymphocytes T ; où ladite leucémie est la leucémie dépendante du HTLV-I, la leucémie lymphocytaire chronique (LLC), la leucémie myélogène aiguë (LMA), la leucémie mégacaryocytaire, la leucémie à grands lymphocytes granuleux (GLG).

12. Composition pour une utilisation selon l'une quelconque des revendications 8 à 11, où ladite affection pathologique est une tumeur résistante à un traitement avec des agents chimiothérapeutiques qui n'inhibent pas STAT3.

13. Kit pour l'administration simultanée ou séquentielle de l'extrait de *Filipendula vulgaris* et d'un ou plusieurs composés dotés d'une activité antitumorale et/ou d'un ou de plusieurs composés dotés d'une activité anti-inflammatoire pour une utilisation dans la prévention et/ou le traitement d'une affection pathologique inflammatoire et/ou pré-tumorale et/ou tumorale **caractérisée par** une activation constitutive ou anormale du facteur de transcription STAT3, comprenant une ou plusieurs aliquotes d'un extrait de *Filipendula vulgaris* tel que défini dans les revendications 1 ou 2, et une ou plusieurs aliquotes d'un ou de plusieurs composés antitumoraux et/ou une ou plusieurs aliquotes d'un ou de plusieurs composés anti-inflammatoires.

14. Kit selon la revendication 13, dans lequel lesdits un ou plusieurs composés antitumoraux sont choisis dans le groupe comprenant : le cisplatine, la doxorubicine, le pémétrexed, le méthotrexate, la vinorelbine, la gemcitabine et le taxol.

15. Kit selon l'une quelconque des revendications 17 ou 18, où ladite affection pathologique est une affection pathologique tumorale choisie dans le groupe comprenant : le cancer de la prostate, le myélome multiple, la leucémie, le lymphome, le mélanome, le carcinome de l'ovaire, le carcinome des cellules rénales, l'adénocarcinome pancréatique, le cancer du poumon, le cancer du cerveau, l'érythroleucémie, les carcinomes à cellules squameuses de la tête et du cou, le cancer du côlon, le mésothéliome.
